(19) Europäisches Patentamt
European Patent Office
Office européen des brevets

(11) EP 1 314 784 A1

(12) EUROPEAN PATENT APPLICATION

(43) Date of publication:
**28.05.2003 Bulletin 2003/22**

(21) Application number: **01127439.6**

(22) Date of filing: **27.11.2001**

(51) Int Cl.[7]: **C12N 15/12**, C12N 1/21, C12N 5/10, C12N 15/63, C07K 14/705, C12Q 1/68, G01N 33/68, A61K 31/7088, A61K 31/713, A61K 38/17, A61K 39/395, A61K 48/00

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **BAYER AG**
**51368 Leverkusen (DE)**

(72) Inventors:
- **Golz, Stefan, Dr.**
  **45326 Essen (DE)**
- **Brüggemeier, Ulf, Dr.**
  **42799 Leichlingen (DE)**
- **Geerts, Andreas, Dipl.-Biol.**
  **42113 Wuppertal (DE)**

(54) **G-protein coupled receptor LUSTR2 and uses thereof**

(57) Polynucleotide sequence for novel G-protein coupled receptor LUSTR2 and uses thereof.

The present invention is directed to a polynucleotide sequence of a novel G-Protein Coupled Receptor LUSTR2. More particularly, the present invention provides a polynucleotide sequence comprising the nucleic acid sequence SEQ ID NO: 1 or nucleic acid sequences that hybridize to SEQ ID NO: 1 or its complimentary strand having at least 40% sequence identity. Preferably, the hybridizing nucleic acid sequence should hybridize under stringent conditions and in particular have 85% sequence identity, more preferably 90% sequence identity, and most preferably 95% sequence identity.

EP 1 314 784 A1

SEQUENCE LISTING

<110> Bayer AG

Schmidt, Karsten

<120> New G-protein coupled receptor LUSTR2 and uses thereof

<130> LeA 35777

<160> 5

<170> PatentIn version 3.1

<210> 1

<211> 1632

<212> DNA

<213> Homo sapiens

<400> 1

```
atggcagtga gcgagaggag ggggctcggc cgcgggagcc ccgcggagtg ggggcagcgg      60
ctacttctgg tgctgctgtt gggtggctgc tccgggcgca tccaccggct ggcgctgacg     120
ggggagaagc gagcggacat ccagctgaac agcttcggtt tctacaccaa tggctctctg     180
gaggtggagt tgagcgtcct gcggctgggc ctccgggagg cagaagagaa gtccctgctg     240
gtggggttca gtctcagccg ggttcggtct ggcagagttc gctcctattc aacccgggat     300
ttccaggact gccctctcca gaaaaacagt agcagtttcc tggtcctgtt cctcatcaac     360
accaaggatc tgcaggtcca ggtgcggaag tatggagagc agaagacgtt gtttatcttt     420
cccgggctcc tcccggaagc accctccaaa ccagggctcc cgaagccaca ggccacagtc     480
ccccgcaagg tggatggcgg agggacctct gcagccagca agcccaagtc aacacccgca     540
gtgattcagg gtcctagtgg gaaggacaag gacctggtgt tgggcctgag ccacctcaac     600
```

aactcctaca acttcagttt ccacgtggtg atcggctctc aggcggaaga aggccagtac 660

agcctgaact tccacaactg caacaattca gtgccaggaa aggagcatcc attcgacatc 720

acggtgatga tccgggagaa gaaccccgat ggcttcctgt cggcagcgga gatgcccctt 780

ttcaagctct acatggtcat gtccgcctgc ttcctggccg ctggcatctt ctgggtgtcc 840

atcctctgca ggaacacgta cagcgtcttc aagatccact ggctcatggc ggccttggcc 900

ttcaccaaga gcatctctct cctcttccac agcatcaact actacttcat caacagccag 960

ggccacccca tcgaaggcct tgccgtcatg tactacatcg cacacctgct gaagggcgcc 1020

ctcctcttca tcaccatcgc cctgattggc tcaggctggg ccttcatcaa gtacgtcctg 1080

tcggataagg agaagaaggt ctttgggatc gtgatcccca tgcaggtcct ggccaacgtg 1140

gcctacatca tcatcgagtc ccgcgaggaa ggcgccagcg actacgtgct gtggaaggag 1200

atttgttcc tggtggacct catctgctgt ggtgccatcc tgttccccgt agtctggtcc 1260

atccggcatc tccaggatgc gtctggcaca gacgggaagg tggcagtgaa cctggccaag 1320

ctgaagctgt tccggcatta ctatgtcatg gtcatctgct acgtctactt cacccgcatc 1380

atcgccatcc tgctgcaggt ggctgtgccc tttcagtggc agtggctgta ccagctcttg 1440

gtggagggct ccaccctggc cttcttcgtg ctcacgggct acaagttcca gcccacagga 1500

aacaacccgt acctgcagct gccccaggag gacgaggagg atgttcagat ggagcaagta 1560

atgacggact ctgggttccg ggaaggcctc tccaaagtca acaaaacagc cagcgggcgg 1620

gaactgttat ga 1632

<210> 2

<211> 543

<212> PRT

<213> Homo sapiens

<400> 2

Met Ala Val Ser Glu Arg Arg Gly Leu Gly Arg Gly Ser Pro Ala Glu
1 5 10 15

Trp Gly Gln Arg Leu Leu Leu Val Leu Leu Leu Gly Gly Cys Ser Gly
20 25 30

```
Arg Ile His Arg Leu Ala Leu Thr Gly Glu Lys Arg Ala Asp Ile Gln
        35                  40                  45

Leu Asn Ser Phe Gly Phe Tyr Thr Asn Gly Ser Leu Glu Val Glu Leu
    50                  55                  60

Ser Val Leu Arg Leu Gly Leu Arg Glu Ala Glu Glu Lys Ser Leu Leu
65                  70                  75                  80

Val Gly Phe Ser Leu Ser Arg Val Arg Ser Gly Arg Val Arg Ser Tyr
                85                  90                  95

Ser Thr Arg Asp Phe Gln Asp Cys Pro Leu Gln Lys Asn Ser Ser Ser
            100                 105                 110

Phe Leu Val Leu Phe Leu Ile Asn Thr Lys Asp Leu Gln Val Gln Val
        115                 120                 125

Arg Lys Tyr Gly Glu Gln Lys Thr Leu Phe Ile Phe Pro Gly Leu Leu
    130                 135                 140

Pro Glu Ala Pro Ser Lys Pro Gly Leu Pro Lys Pro Gln Ala Thr Val
145                 150                 155                 160

Pro Arg Lys Val Asp Gly Gly Gly Thr Ser Ala Ala Ser Lys Pro Lys
                165                 170                 175

Ser Thr Pro Ala Val Ile Gln Gly Pro Ser Gly Lys Asp Lys Asp Leu
            180                 185                 190

Val Leu Gly Leu Ser His Leu Asn Asn Ser Tyr Asn Phe Ser Phe His
        195                 200                 205

Val Val Ile Gly Ser Gln Ala Glu Glu Gly Gln Tyr Ser Leu Asn Phe
    210                 215                 220

His Asn Cys Asn Asn Ser Val Pro Gly Lys Glu His Pro Phe Asp Ile
225                 230                 235                 240

Thr Val Met Ile Arg Glu Lys Asn Pro Asp Gly Phe Leu Ser Ala Ala
                245                 250                 255

Glu Met Pro Leu Phe Lys Leu Tyr Met Val Met Ser Ala Cys Phe Leu
```

```
                260                 265                 270

Ala Ala Gly Ile Phe Trp Val Ser Ile Leu Cys Arg Asn Thr Tyr Ser
        275                 280                 285

Val Phe Lys Ile His Trp Leu Met Ala Ala Leu Ala Phe Thr Lys Ser
    290                 295                 300

Ile Ser Leu Leu Phe His Ser Ile Asn Tyr Tyr Phe Ile Asn Ser Gln
305                 310                 315                 320

Gly His Pro Ile Glu Gly Leu Ala Val Met Tyr Tyr Ile Ala His Leu
                325                 330                 335

Leu Lys Gly Ala Leu Leu Phe Ile Thr Ile Ala Leu Ile Gly Ser Gly
            340                 345                 350

Trp Ala Phe Ile Lys Tyr Val Leu Ser Asp Lys Glu Lys Lys Val Phe
        355                 360                 365

Gly Ile Val Ile Pro Met Gln Val Leu Ala Asn Val Ala Tyr Ile Ile
    370                 375                 380

Ile Glu Ser Arg Glu Glu Gly Ala Ser Asp Tyr Val Leu Trp Lys Glu
385                 390                 395                 400

Ile Leu Phe Leu Val Asp Leu Ile Cys Cys Gly Ala Ile Leu Phe Pro
                405                 410                 415

Val Val Trp Ser Ile Arg His Leu Gln Asp Ala Ser Gly Thr Asp Gly
            420                 425                 430

Lys Val Ala Val Asn Leu Ala Lys Leu Lys Leu Phe Arg His Tyr Tyr
        435                 440                 445

Val Met Val Ile Cys Tyr Val Tyr Phe Thr Arg Ile Ile Ala Ile Leu
    450                 455                 460

Leu Gln Val Ala Val Pro Phe Gln Trp Gln Trp Leu Tyr Gln Leu Leu
465                 470                 475                 480

Val Glu Gly Ser Thr Leu Ala Phe Phe Val Leu Thr Gly Tyr Lys Phe
                485                 490                 495
```

Gln Pro Thr Gly Asn Asn Pro Tyr Leu Gln Leu Pro Gln Glu Asp Glu
500 505 510

Glu Asp Val Gln Met Glu Gln Val Met Thr Asp Ser Gly Phe Arg Glu
515 520 525

Gly Leu Ser Lys Val Asn Lys Thr Ala Ser Gly Arg Glu Leu Leu
530 535 540

<210> 3

<211> 19

<212> DNA

<213> Artificial

<400> 3
gggttcggtc tggcagagt 19

<210> 4

<211> 25

<212> DNA

<213> Artificial

<400> 4
cgctcctatt caacccggga tttcc 25

<210> 5

<211> 21

<212> DNA

<213> Artificial

<400> 5
tttttctgga gagggcagtc c 21

## Description

### Technical field of the invention

The present invention is in the field of molecular biology; more particularly, the present invention describes a nucleic acid sequence and an amino acid sequence for a novel human LUSTR2 and its regulation for therapeutic purposes.

### Background of the invention

### G-Protein Coupled Receptors

The LUSTR2 is a seven transmembrane G protein coupled receptor (GPCR). Many medically significant biological processes are mediated by signal transduction pathways that involve G-proteins. [Lefkowitz et al. 1991)] . The family of G-protein coupled receptors (GPCR) includes receptors for hormones, neurotransmitters, growth factors, and viruses. Specific examples of GPCRs include receptors for such diverse agents as dopamine, calcitonine, adrenergic hormones, endotheline, cAMP, adenosine, acetylcholine, serotonine, histamine, thrombin, kinine, follicle stimulating hormone, opsins, endothelial differentiation gene-1, rhodopsins, odorants, cytomegalovirus, G-proteins themselves, effector proteins such as phospholipase C, adenyl cyclase, and phosphodiesterase, and actuator proteins such as protein kinase A and protein kinase C.

GPCRs possess seven conserved membrane-spanning domains connecting at least eight divergent hydrophilic loops. GPCRs, also known as seven trans-membrane, 7TM, receptors, have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 is being implicated with signal transduction. Phosphorylation and lipidation (palmitylation or farnesylation) of cysteine residues can influence signal transduction of some GPCRs. Most GPCRs contain potential phosphorylation sites within the third cytoplasmic loop and/or the carboxy terminus. For several GPCRs, such as the beta-adrenergic receptor, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 is being implicated with several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters. Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation of the enzyme, adenylate cyclase, inside the cell. Enzyme activation by hormones is dependent on the presence of the nucleotide GTP. GTP also influences hormone binding. A G-protein connects the hormone receptor to adenylate cyclase. G-protein exchanges GTP for bound GDP when activated by a hormone receptor. The GTP-carrying form then binds to activated adenylate cyclase. Hydrolysis of GTP to GDP, catalyzed by the G-protein itself, returns the G-protein to its basal, inactive form. Thus, the G-protein serves a dual role, as an intermediate that relays the signal from receptor to effector, and as a clock that controls the duration of the signal.

Over the past 15 years, nearly 350 therapeutic agents targeting 7TM receptors have been successfully introduced into the market. This indicates that these receptors have an established, proven history as therapeutic targets. Clearly, there is a need for identification and characterization of further receptors which can play a role in preventing, ameliorating, or correcting dysfunctions or diseases including, but not limited to, infections such as bacterial, fungal, protozoan, and viral infections, particularly those caused by HIV viruses, cancers, allergies including asthma, cardiovascular diseases including acute heart failure, hypotension, hypertension, angina pectoris, myocardial infarction, haematological diseases, genito-urinary diseases including urinary incontinence and benign prostate hyperplasia, osteoporosis, and peripheral and central nervous system disorders including pain, Alzheimer's disease and Parkinson's disease.

**TaqMan-Technology / human tissue localisation**

TaqMan is a recently developed technique, in which the release of a fluorescent reporter dye from a hybridisation probe in real-time during a polymerase chain reaction (PCR) is proportional to the accumulation of the PCR product. Quantification is based on the early, linear part of the reaction, and by determining the threshold cycle (CT), at which fluorescence above background is first detected.

Gene expression technologies may be useful in several areas of drug discovery and development, such as target identification, lead optimization, and identification of mechanisms of action. The TaqMan technology can be used to compare differences between expression profiles of normal tissue and diseased tissue. Expression profiling has been used in identifying genes, which are up- or downregulated in a variety of diseases. An interesting application of expression profiling is temporal monitoring of changes in gene expression during disease progression and drug treatment or in patients versus healthy individuals. The premise in this approach is that changes in pattern of gene expression in response to physiological or environmental stimuli (e.g. drugs) may serve as indirect clues about disease-causing genes or drug targets. Moreover, the effects of drugs with established efficacy on global gene expression patterns may provide a guidepost, or a genetic signature, against which a new drug candidate can be compared.

**Summary of the invention**

The invention relates to a nucleotide sequence which encodes a novel human LUSTR2. In the following LUSTR2 designates a polypeptide having the sequence of or being homologous to SEQ ID NO:2, and having LUSTR2 activity. LUSTR2 further contemplates various polypeptides arising from post-translational modifications of the polypeptide including but not limited to acetylation, carboxylation, glycosylation, phosphorylation, lipidation and acylation. The invention relates to nucleic acid molecules encoding LUSTR2 and polypeptides having LUSTR2-activity, and to their use in the diagnosis or treatment of diseases associated with expression of LUSTR2.

It is an object of the invention to provide reagents and methods for regulating the expression and activity of human LUSTR2 for the treatment of haematological disorders, disorders of the peripheral and central nervous system, COPD, asthma, genito-urological disorders, metabolic diseases and pancreas and heart disorders. This and other objects of the invention are provided by one or more of the embodiments described below.

Another object of the invention is a method of screening for agents which can regulate the activity of LUSTR2. A test compound is contacted with a polypeptide comprising the amino acid sequence selected of the group consisting of SEQ ID NO:2 or a polypeptide which exhibits LUSTR2 activity and is encoded by a polynucleotide hybridizing under stringent conditions to polynucleotide shown in SEQ ID NO:1; and binding of the test compound to LUSTR2 is detected, wherein a test compound which binds to the polypeptide is identified as a potential therapeutic agent for decreasing the activity of LUSTR2. Another embodiment of the invention is a method of screening for agents which can regulate the activity of LUSTR2. A test compound contacted with a polypeptide comprising the amino acid sequence selected from a group consisting of SEQ ID NO:2 or a polypeptide which exhibits LUSTR2 activity and is encoded by a polynucleotide hybridizing under stringent conditions to polynucleotide shown in SEQ ID NO:1; and LUSTR2 activity of the polypeptide is detected, wherein a test compound which increases LUSTR2 activity is identified as a potential therapeutic agent for increasing the activity of LUSTR2, and wherein a test compound which decreases LUSTR2 activity of the polypeptide is identified as a potential therapeutic agent for decreasing the activity of LUSTR2.

Another object of the invention is a method of screening for agents which can regulate the activity of LUSTR2. A test compound is contacted with a polynucleotide comprising the sequence selected of the group consisting of (1) SEQ ID NO:1 or (2) a polynucleotide which encodes a polypeptide exhibiting LUSTR2 activity and hybridizes under stringent conditions to the polynucleotide shown in SEQ ID NO:1; and binding of the test compound to the polynucleotide is detected, wherein a test compound which binds to the polynucleotide is identified as a potential therapeutic agent for decreasing the activity of LUSTR2.

Another object of the invention is a method of screening for agents which can regulate the activity of LUSTR2. A test compound is contacted with a product encoded by a polynucleotide which comprises the nucleotide sequence shown in SEQ ID NO:1; and binding of the test compound to the product is detected, wherein a test compound which binds to the product is identified as a potential agent for regulating the activity of LUSTR2.

Another object of the invention is a method of reducing the activity of LUSTR2. A cell is contacted with a reagent which specifically binds to a polynucleotide encoding LUSTR2 or the LUSTR2 polypeptide. LUSTR2 activity is thereby reduced.

Another object of the invention is a method of increasing the activity of LUSTR2. A cell is contacted with a reagent which specifically binds to a polynucleotide encoding LUSTR2 or the LUSTR2 polypeptide. LUSTR2 activity is thereby increased.

Another object of the invention is the antisense DNA of DNA encoding LUSTR2; cloning or expression vectors containing nucleic acid encoding LUSTR2; host cells or organisms transformed with expression vectors containing nucleic acid encoding LUSTR2; a method for the production and recovery of purified LUSTR2 from host cells: purified protein, LUSTR2, which can be used to identify inhibitors or activators of signal transduction involving LUSTR2; and methods of screening for ligands of LUSTR2 using transformed cells.

## Brief Description of the Drawings

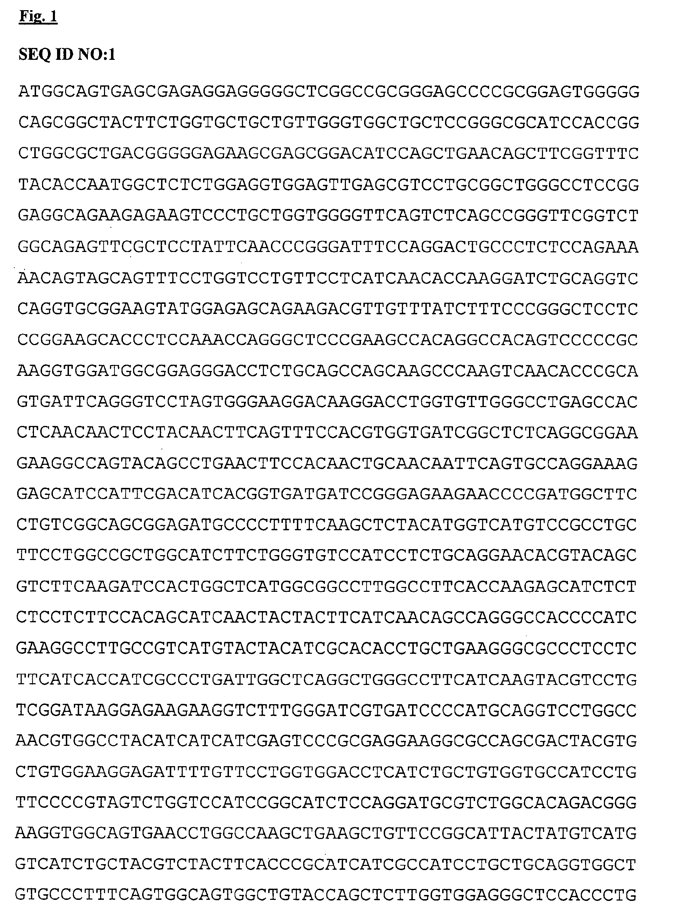

Fig. 1 shows the nucleotide sequence of a LUSTR2 polynucleotide (SEQ ID NO:1).

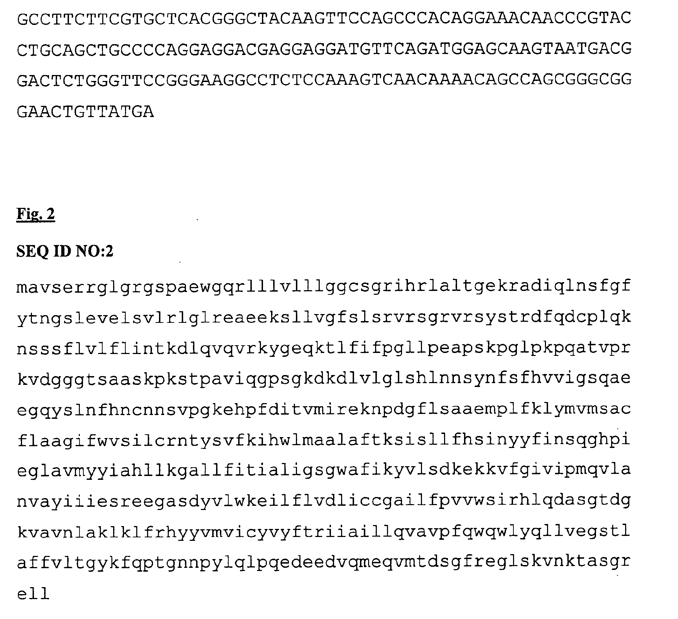

Fig. 2 shows the amino acid sequence of a LUSTR2 polypolypeptide (SEQ ID NO:2).

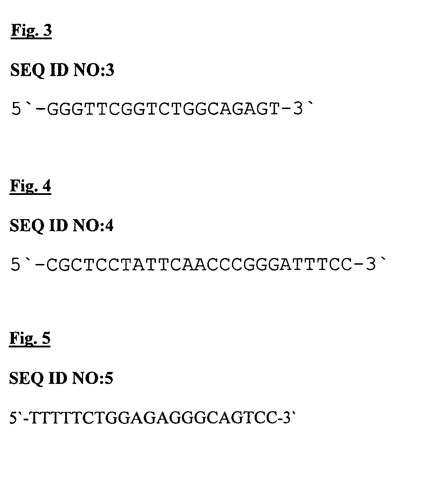

Fig. 3 shows the nucleotide sequence of a primer useful for the invention (SEQ ID NO:3).
Fig. 4 shows the nucleotide sequence of a primer useful for the invention (SEQ ID NO:4).
Fig. 5 shows the nucleotide sequence of a primer useful for the invention (SEQ ID NO:5).

## Detailed description of the invention

### Nucleotide sequence

As used herein and designated by the upper case abbreviation, LUSTR2, refers to an GPCR in either naturally occurring or synthetic form and active fragments thereof which have the amino acid sequence of SEQ. ID NO:2. In one embodiment, the polypeptide LUSTR2 is encoded by mRNAs transcribed from the cDNA, as designated by the lower case abbreviation, LUSTR2, of SEQ. ID NO:1.

The novel human LUSTR2 receptor shows a homology of 87% to the mus musculus receptor LUSTR2 (Genbank : AF376726). The sequence of LUSTR2 was assembled from EST and genomic sequences (AL365404.1; XM_044591.1).

An "oligonucleotide" is a stretch of nucleotide residues which has a sufficient number of bases to be used as an oligomer, amplimer or probe in a polymerase chain reaction (PCR). Oligonucleotides are prepared from genomic or cDNA sequence and are used to amplify, reveal, or confirm the presence of a similar DNA or RNA in a particular cell or tissue. Oligonucleotides or oligomers comprise portions of a DNA sequence having at least about 10 nucleotides and as many as about 35 nucleotides, preferably about 25 nucleotides.

"Probes" may be derived from naturally occurring or recombinant single- or double-stranded nucleic acids or may be chemically synthesized. They are useful in detecting the presence of identical or similar sequences. Such probes may be labeled with reporter molecules using nick translation, Klenow fill-in reaction, PCR or other methods well known in the art. Nucleic acid probes may be used in southern, northern or in situ hybridizations to determine whether DNA or RNA encoding a certain protein is present in a cell type, tissue, or organ.

A fragment of a polynucleotide or nucleic acid that comprises all or any part of the nucleotide sequence having fewer nucleotides than about 6 kb, preferably fewer than about 1 kb which can be used as a probe.

"Reporter" molecules are radionuclides, enzymes, fluorescent, chemiluminescent, or chromogenic agents which associate with a particular nucleotide or amino acid sequence, thereby establishing the presence of a certain sequence, or allowing for the quantification of a certain sequence.

"Recombinant nucleotide variants" encoding LUSTR2 may be synthesized by making use of the "redundancy" in the genetic code. Various codon substitutions, such as the silent changes which produce specific restriction sites or codon usage-specific mutations, may be introduced to optimize cloning into a plasmid or viral vector or expression in a particular prokaryotic or eukaryotic host system, respectively.

"Chimeric" molecules may be constructed by introducing all or part of the nucleotide sequence of this invention into a vector containing additional nucleic acid sequence which might be expected to change any one or several of the following LUSTR2 characteristics: cellular location, distribution, ligand-binding affinities, interchain affinities, degradation/turnover rate, signaling, etc.

"Active" refers to those forms, fragments, or domains of LUSTR2 which retain the biological and/or antigenic activities of LUSTR2.

"Naturally occurring LUSTR2" refers to a polypeptide produced by cells which have not been genetically engineered and specifically contemplates various polypeptides arising from post-translational modifications of the polypeptide including but not limited to acetylation, carboxylation, glycosylation, phosphorylation, lipidation and acylation.

"Derivative" refers to polypeptides which have been chemically modified by techniques such as ubiquitination, labeling (see above), pegylation (derivatization with polyethylene glycol), and chemical insertion or substitution of amino acids such as ornithine which do not normally occur in human proteins.

"Recombinant polypeptide variant" refers to any polypeptide which differs from naturally occurring LUSTR2 by amino acid insertions, deletions and/or substitutions, created using recombinant DNA techniques. Guidance in determining which amino acid residues may be replaced, added, or deleted, without abolishing activities of interest may be found by comparing the sequence of the polypeptide of interest with that of related polypeptides and minimizing the number of amino acid sequence changes made in highly conserved regions.

Conservative Amino acid "substitutions" result from replacing one amino acid with another having similar structural and/or chemical properties, such as the replacement of a leucine with an isoleucine or valine, an aspartate with a glutamate, or a threonine with a serine.

"Insertions" or "deletions" are typically in the range of about 1 to 5 amino acids. The variation allowed may be experimentally determined by producing the peptide synthetically while systematically making insertions, deletions, or substitutions of nucleotides in the sequence using recombinant DNA techniques.

A "signal or leader sequence" can be used, when desired, to direct the polypeptide through a membrane of a cell. Such a sequence may be naturally present on the polypeptides of the present invention or provided from heterologous sources by recombinant DNA techniques.

An "oligopeptide" is a short stretch of amino acid residues and may be expressed from an oligonucleotide. Oligopeptides comprise a stretch of amino acid residues of at least 3, 5, 10 amino acids and at most 10, 15, 25 amino acids, typically of at least 9 to 13 amino acids, and of sufficient length to display biological and/or antigenic activity.

" Inhibitor" is any substance which retards or prevents a chemical or physiological reaction or response. Common inhibitors include but are not limited to antisense molecules, antibodies, and antagonists.

"Standard" expression is a quantitative or qualitative measurement for comparison. It is based on a statistically appropriate number of normal samples and is created to use as a basis of comparison when performing diagnostic assays, running clinical trials, or following patient treatment profiles.

"Animal" as used herein may be defined to include human, domestic (cats dogs, etc.), agricultural (cows, horses, sheep, etc.) or test species (mouse, rat, rabbit, etc.).

The nucleotide sequences encoding LUSTR2 (or their complement) have numerous applications in techniques known to those skilled in the art of molecular biology. These techniques include use as hybridization probes, use in the construction of oligomers for PCR, use for chromosome and gene mapping, use in the recombinant production of LUSTR2, and use in generation of antisense DNA or RNA, their chemical analogs and the like. Uses of nucleotides encoding LUSTR2 disclosed herein are exemplary of known techniques and are not intended to limit their use in any technique known to a person of ordinary skill in the art. Furthermore, the nucleotide sequences disclosed herein may be used in molecular biology techniques that have not yet been developed, provided the new techniques rely on properties of nucleotide sequences that are currently known, e.g., the triplet genetic code, specific base pair interactions, etc.

It will be appreciated by those skilled in the art that as a result of the degeneracy of the genetic code, a multitude of LUSTR2 -encoding nucleotide sequences may be produced. Some of these will only bear minimal homology to the nucleotide sequence of the known and naturally occurring LUSTR2. The invention has specifically contemplated each and every possible variation of nucleotide sequence that could be made by selecting combinations based on possible codon choices. These combinations are made in accordance with the standard triplet genetic code as applied to the nucleotide sequence of naturally occurring LUSTR2, and all such variations are to be considered as being specifically disclosed.

Although the nucleotide sequences which encode LUSTR2, its derivatives or its variants are preferably capable of hybridizing to the nucleotide sequence of the naturally occurring LUSTR2 under stringent conditions, it may be advantageous to produce nucleotide sequences encoding LUSTR2 or its derivatives possessing a substantially different codon usage. Codons can be selected to increase the rate at which expression of the peptide occurs in a particular prokaryotic or eukaryotic expression host in accordance with the frequency with which particular codons are utilized by the host. Other reasons for substantially altering the nucleotide sequence encoding LUSTR2 and/or its derivatives without altering the encoded amino acid sequence include the production of RNA transcripts having more desirable properties, such as a greater half-life, than transcripts produced from the naturally occurring sequence.

Nucleotide sequences encoding LUSTR2 may be joined to a variety of other nucleotide sequences by means of well established recombinant DNA techniques. Useful nucleotide sequences for joining to LUSTR2 include an assortment of cloning vectors such as plasmids, cosmids, lambda phage derivatives, phagemids, and the like. Vectors of interest include expression vectors, replication vectors, probe generation vectors, sequencing vectors, etc. In general, vectors of interest may contain an origin of replication functional in at least one organism, convenient restriction endonuclease sensitive sites, and selectable markers for one or more host cell systems.

Another aspect of the subject invention is to provide for LUSTR2-specific hybridization probes capable of hybridizing with naturally occurring nucleotide sequences encoding LUSTR2. Such probes may also be used for the detection of similar GPCR encoding sequences and should preferably contain at least 40% nucleotide identity to LUSTR2 sequence. The hybridization probes of the subject invention may be derived from the nucleotide sequence presented as SEQ. ID NO: 1 or from genomic sequences including promoter, enhancers or introns of the native gene. Hybridization probes may be labeled by a variety of reporter molecules using techniques well known in the art.

It will be recognized that many deletional or mutational analogs of nucleic acid sequences for LUSTR2 will be effective hybridization probes for LUSTR2 nucleic acid. Accordingly, the invention relates to nucleic acid sequences that hybridize with such LUSTR2 encoding nucleic acid sequences under stringent conditions.

"Stringent conditions" refers to conditions that allow for the hybridization of substantially related nucleic acid sequences. For instance, such conditions will generally allow hybridization of sequence with at least about 85% sequence identity, preferably with at least about 90% sequence identity, more preferably with at least about 95% sequence identity. Hybridization conditions and probes can be adjusted in well-characterized ways to achieve selective hybridization of human-derived probes.

Nucleic acid molecules that will hybridize to LUSTR2 encoding nucleic acid under stringent conditions can be identified functionally. Without limitation, examples of the uses for hybridization probes include: histochemical uses such as identifying tissues that express LUSTR2; measuring mRNA levels, for instance to identify a sample's tissue type or to identify cells that express abnormal levels of LUSTR2; and detecting polymorphisms in LUSTR2.

PCR as described in U.S. Pat. Nos. 4,683,195; 4,800,195; and 4,965,188 provides additional uses for oligonucleotides based upon the nucleotide sequence which encodes LUSTR2. Such probes used in PCR may be of recombinant origin, chemically synthesized, or a mixture of both. Oligomers may comprise discrete nucleotide sequences employed under optimized conditions for identification of LUSTR2 in specific tissues or diagnostic use. The same two oligomers, a nested set of oligomers, or even a degenerate pool of oligomers may be employed under less stringent conditions for identification of closely related DNAs or RNAs.

Rules for designing polymerase chain reaction ("PCR") primers are now established, as reviewed by PCR Protocols [Devlin et al]. Degenerate primers, i.e., preparations of primers that are heterogeneous at given sequence locations, can be designed to amplify nucleic acid sequences that are highly homologous to, but not identical with LUSTR2. Strategies are now available that allow for only one of the primers to be required to specifically hybridize with a known sequence. For example, appropriate nucleic acid primers can be ligated to the nucleic acid sought to be amplified to provide the hybridization partner for one of the primers. In this way, only one of the primers need be based on the sequence of the nucleic acid sought to be amplified.

PCR methods for amplifying nucleic acid will utilize at least two primers. One of these primers will be capable of hybridizing to a first strand of the nucleic acid to be amplified and of priming enzyme-driven nucleic acid synthesis in a first direction. The other will be capable of hybridizing the reciprocal sequence of the first strand (if the sequence to be amplified is single stranded, this sequence will initially be hypothetical, but will be synthesized in the first amplification cycle) and of priming nucleic acid synthesis from that strand in the direction opposite the first direction and towards the site of hybridization for the first primer. Conditions for conducting such amplifications, particularly under preferred stringent hybridization conditions, are well known.

Other means of producing specific hybridization probes for LUSTR2 include the cloning of nucleic acid sequences encoding LUSTR2 or LUSTR2 derivatives into vectors for the production of mRNA probes. Such vectors are known in the art, are commercially available and may be used to synthesize RNA probes in vitro by means of the addition of the appropriate RNA polymerase as T7 or SP6 RNA polymerase and the appropriate reporter molecules.

It is possible to produce a DNA sequence, or portions thereof, entirely by synthetic chemistry. After synthesis, the nucleic acid sequence can be inserted into any of the many available DNA vectors and their respective host cells using techniques which are well known in the art. Moreover, synthetic chemistry may be used to introduce mutations into the nucleotide sequence. Alternately, a portion of sequence in which a mutation is desired can be synthesized and recombined with longer portion of an existing genomic or recombinant sequence.

Nucleotide sequences encoding LUSTR2 may be used to produce a purified oligo-or polypeptide using well known methods of recombinant DNA technology. The oligopeptide may be expressed in a variety of host cells, either prokaryotic or eukaryotic. Host cells may be from the same species from which the nucleotide sequence was derived or from a different species. Advantages of producing an oligonucleotide by recombinant DNA technology include obtaining adequate amounts of the protein for purification and the availability of simplified purification procedures.

**Quantitative determinations of nucleic acids**

An important step in the molecular genetic analysis of human disease is often the enumeration of the copy number of a nucleis acid or the relative expression of a gene in particular tissues.

Several different approaches are currently available to make quantitative determinations of nucleic acids. Chromosome-based techniques, such as comparative genomic hybridization (CGH) and fluorescent in situ hybridization (FISH) facilitate efforts to cytogenetically localize genomic regions that are altered in tumor cells. Regions of genomic alteration can be narrowed further using loss of heterozygosity analysis (LOH), in which disease DNA is analyzed and compared with normal DNA for the loss of a heterozygous polymorphic marker. The first experiments used restriction fragment length polymorphisms (RFLPs) [Johnson et al], or hypervariable minisatellite DNA [Barnes et al] In recent years LOH has been performed primarily using PCR amplification of microsatellite markers and electrophoresis of the radiolabeled [Jeffreys et al] or fluorescently labeled PCR products [Weber et al] and compared between paired normal and disease DNAs.

A number of other methods have also been developed to quantify nucleic acids [Gergen et al, Southern et al, Sharp et al]. More recently, PCR and RT-PCR methods have been developed which are capable of measuring the amount of a nucleic acid in a sample. One approach, for example, measures PCR product quantity in the log phase of the reaction before the formation of reaction products plateaus [Thomas et al].

A gene sequence contained in all samples at relatively constant quantity is typically utilized for sample amplification efficiency normalization. This approach, however, suffers from several drawbacks. The method requires that each sample has equal input amounts of the nucleic acid and that the amplification efficiency between samples is identical until the time of analysis. Furthermore, it is difficult using the conventional methods of PCR quantitation such as gel electrophoresis or plate capture hybridization to determine that all samples are in fact analyzed during the log phase of the reaction as required by the method.

Another method called quantitative competitive (QC)-PCR, as the name implies, relies on the inclusion of an internal control competitor in each reaction [Maniatis et al, Becker-Andre et al , Piatak et al in BioTechniques (1993)] . The efficiency of each reaction is normalized to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency than the target molecule.

*5' Fluorogenic Nuclease Assays*

Fluorogenic nuclease assays are a real time quantitation method that uses a probe to monitor formation of amplification product. The basis for this method of monitoring the formation of amplification product is to measure continuously PCR product accumulation using a dual-labeled fluorogenic oligonucleotide probe, an approach frequently referred to in the literature simply as the "TaqMan method" [Piatak et al. in Science(1993), Heid et al., Gibson et al, Holland et al.].

The probe used in such assays is typically a short (about 20-25 bases) oligonucleotide that is labeled with two different fluorescent dyes. The 5' terminus of the probe is attached to a reporter dye and the 3' terminus is attached to a quenching dye, although the dyes could be attached at other locations on the probe as well. The probe is designed to have at least substantial sequence complementarity with the probe binding site. Upstream and downstream PCR primers which bind to flanking regions of the locus are added to the reaction mixture. When the probe is intact, energy transfer between the two fluorophors occurs and the quencher quenches emission from the reporter. During the extension phase of PCR, the probe is cleaved by the 5' nuclease activity of a nucleic acid polymerase such as Taq polymerase, thereby releasing the reporter from the oligonucleotide-quencher and resulting in an increase of reporter emission intensity which can be measured by an appropriate detector.

One detector which is specifically adapted for measuring fluorescence emissions such as those created during a fluorogenic assay is the ABI 7700 or 4700 HT manufactured by Applied Biosystems, Inc. in Foster City, Calif. The ABI 7700 uses fiber optics connected with each well in a 96-or 384 well PCR tube arrangement. The instrument includes a laser for exciting the labels and is capable of measuring the fluorescence spectra intensity from each tube with continuous monitoring during PCR amplification. Each tube is reexamined every 8.5 seconds.

Computer software provided with the instrument is capable of recording the fluorescence intensity of reporter and quencher over the course of the amplification. The recorded values will then be used to calculate the increase in normalized reporter emission intensity on a continuous basis. The increase in emission intensity is plotted versus time, i.e., the number of amplification cycles, to produce a continuous measure of amplification. To quantify the locus in each amplification reaction, the amplification plot is examined at a point during the log phase of product accumulation. This is accomplished by assigning a fluorescence threshold intensity above background and determining the point at which each amplification plot crosses the threshold (defined as the threshold cycle number or Ct). Differences in threshold cycle number are used to quantify the relative amount of PCR target contained within each tube. Assuming that each reaction functions at 100% PCR efficiency, a difference of one Ct represents a two-fold difference in the amount of starting template. The fluorescence value can be used in conjunction with a standard curve to determine the amount of amplification product present.

*Non-Probe-Based Detection Methods*

A variety of options are available for measuring the amplification products as they are formed. One method utilizes labels, such as dyes, which only bind to double stranded DNA. In this type of approach, amplification product (which is double stranded) binds dye molecules in solution to form a complex. With the appropriate dyes, it is possible to distinguish between dye molecules free in solution and dye molecules bound to amplification product. For example, certain dyes fluoresce only when bound to amplification product. Examples of dyes which can be used in methods of this general type include, but are not limited to, Syber Green.TM. and Pico Green from Molecular Probes, Inc. of Eugene, Oreg., ethidium bromide, propidium iodide, chromomycin, acridine orange, Hoechst 33258, Toto-1, Yoyo-1, DAPI (4',6-diamidino-2-phenylindole hydrochloride).

Another real time detection technique measures alteration in energy fluorescence energy transfer between fluorophors conjugated with PCR primers [Livak et al.].

*Probe-Based Detection Methods*

These detection methods involve some alteration to the structure or conformation of a probe hybridized to the locus between the amplification primer pair. In some instances, the alteration is caused by the template-dependent extension catalyzed by a nucleic acid polymerase during the amplification process. The alteration generates a detectable signal which is an indirect measure of the amount of amplification product formed.

For example, some methods involve the degradation or digestion of the probe during the extension reaction. These methods are a consequence of the 5'-3' nuclease activity associated with some nucleic acid polymerases. Polymerases having this activity cleave mononucleotides or small oligonucleotides from an oligonucleotide probe annealed to its complementary sequence located within the locus.

The 3' end of the upstream primer provides the initial binding site for the nucleic acid polymerase. As the polymerase catalyzes extension of the upstream primer and encounters the bound probe, the nucleic acid polymerase displaces a portion of the 5' end of the probe and through its nuclease activity cleaves mononucleotides or oligonucleotides from the probe.

The upstream primer and the probe can be designed such that they anneal to the complementary strand in close proximity to one another. In fact, the 3' end of the upstream primer and the 5' end of the probe may abut one another. In this situation, extension of the upstream primer is not necessary in order for the nucleic acid polymerase to begin cleaving the probe. In the case in which intervening nucleotides separate the upstream primer and the probe, extension of the primer is necessary before the nucleic acid polymerase encounters the 5' end of the probe. Once contact occurs and polymerization continues, the 5'-3' exonuclease activity of the nucleic acid polymerase begins cleaving mononucleotides or oligonucleotides from the 5' end of the probe. Digestion of the probe continues until the remaining portion of the probe dissociates from the complementary strand.

In solution, the two end sections can hybridize with each other to form a hairpin loop. In this conformation, the reporter and quencher dye are in sufficiently close proximity that fluorescence from the reporter dye is effectively quenched by the quencher dye. Hybridized probe, in contrast, results in a linearized conformation in which the extent of quenching is decreased. Thus, by monitoring emission changes for the two dyes, it is possible to indirectly monitor the formation of amplification product.

*Probes*

The labeled probe is selected so that its sequence is substantially complementary to a segment of the test locus or a reference locus. As indicated above, the nucleic acid site to which the probe binds should be located between the primer binding sites for the upstream and downstream amplification primers.

*Primers*

The primers used in the amplification are selected so as to be capable of hybridizing to sequences at flanking regions of the locus being amplified. The primers are chosen to have at least substantial complementarity with the different strands of the nucleic acid being amplified. When a probe is utilized to detect the formation of amplification products, the primers are selected in such that they flank the probe, i.e. are located upstream and downstream of the probe.

The primer must have sufficient length so that it is capable of priming the synthesis of extension products in the presence of an agent for polymerization. The length and composition of the primer depends on many parameters, including, for example, the temperature at which the annealing reaction is conducted, proximity of the probe binding site to that of the primer, relative concentrations of the primer and probe and the particular nucleic acid composition of the probe. Typically the primer includes 15-30 nucleotides. However, the length of the primer may be more or less depending on the complexity of the primer binding site and the factors listed above.

*Labels for Probes and Primers*

The labels used for labeling the probes or primers of the current invention and which can provide the signal corresponding to the quantity of amplification product can take a variety of forms. As indicated above with regard to the 5' fluorogenic nuclease method, a fluorescent signal is one signal which can be measured. However, measurements may also be made, for example, by monitoring radioactivity, colorimetry, absorption, magnetic parameters, or enzymatic activity. Thus, labels which can be employed include, but are not limited to, fluorophors, chromophores, radioactive isotopes, electron dense reagents, enzymes, and ligands having specific binding partners (e.g., biotin-avidin).

Monitoring changes in fluorescence is a particularly useful way to monitor the accumulation of amplification products. A number of labels useful for attachment to probes or primers are commercially available including fluorescein and various fluorescein derivatives such as FAM, HEX, TET and JOE (all which are available from Applied Biosystems, Foster City, Calif.); lucifer yellow, and coumarin derivatives.

Labels may be attached to the probe or primer using a variety of techniques and can be attached at the 5' end, and/or the 3' end and/or at an internal nucleotide. The label can also be attached to spacer arms of various sizes which are attached to the probe or primer. These spacer arms are useful for obtaining a desired distance between multiple labels attached to the probe or primer.

In some instances, a single label may be utilized; whereas, in other instances, such as with the 5' fluorogenic nuclease assays for example, two or more labels are attached to the probe. In cases wherein the probe includes multiple labels, it is generally advisable to maintain spacing between the labels which is sufficient to permit separation of the labels during digestion of the probe through the 5'-3' nuclease activity of the nucleic acid polymerase.

**Patients Exhibiting Symptoms of Disease**

A number of diseases are associated with changes in the copy number of a certain gene. For patients having symptoms of a disease, the real-time PCR method can be used to determine if the patient has copy number alterations which are known to be linked with diseases that are associated with the symptoms the patient has.

**LUSTR2 expression**

*LUSTR2 fusion proteins*

Fusion proteins are useful for generating antibodies against LUSTR2 amino acid sequences and for use in various assay systems. For example, fusion proteins can be used to identify proteins which interact with portions of LUSTR2 peptide. Protein affinity chromatography or library-based assays for protein-protein interactions, such as the yeast two-hybrid or phage display systems, can be used for this purpose. Such methods are well known in the art and also can be used as drug screens.

A LUSTR2 fusion protein comprises two polypeptide segments fused together by means of a peptide bond. The first polypeptide segment can comprise at least 54, 75, 100, 125, 139, 150, 175, 200, 225, 250, or 275 contiguous amino acids of SEQ ID NO:2 or of a biologically active variant, such as those described above. The first polypeptide segment also can comprise full-length LUSTR2.

The second polypeptide segment can be a full-length protein or a protein fragment. Proteins commonly used in fusion protein construction include, but are not limited to β galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, herpes simplex virus (HSV) BP16 protein fusions and G-protein fusions (for example G(alpha)16, Gs, Gi). A fusion protein also can be engineered to contain a cleavage site located adjacent to the LUSTR2.

*Preparation of Polynucleotides*

A naturally occurring LUSTR2 polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated LUSTR2 polynucleotides. For example, restriction enzymes and probes can be used to isolate polynucleotide fragments which comprises LUSTR2 nucleotide sequences. Isolated polynucleotides are in preparations which are free or at least 70, 80, or 90% free of other molecules.

LUSTR2 cDNA molecules can be made with standard molecular biology techniques, using LUSTR2 mRNA as a template. LUSTR2 cDNA molecules can thereafter be replicated using molecular biology techniques known in the art. An amplification technique, such as PCR, can be used to obtain additional copies of polynucleotides of the invention, using either human genomic DNA or cDNA as a template.

Alternatively, synthetic chemistry techniques can be used to synthesizes LUSTR2 polynucleotides. The degeneracy of the genetic code allows alternate nucleotide sequences to be synthesized which will encode LUSTR2 having, for example, an amino acid sequence shown in SEQ ID NO:2 or a biologically active variant thereof.

*Extending Polynucleotides*

Various PCR-based methods can be used to extend nucleic acid sequences encoding human LUSTR2, for example to detect upstream sequences of the LUSTR2 gene such as promoters and regulatory elements. For example, restriction-site PCR uses universal primers to retrieve unknown sequence adjacent to a known locus. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region. Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA. In this method, multiple restriction enzyme digestions and ligations also can be used to place an engineered double-stranded sequence into an unknown fragment of the DNA molecule before performing PCR.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d (T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate equipment and software (*e.g.* GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

*Obtaining Polypeptides*

LUSTR2 can be obtained, for example, by purification from human cells, by expression of LUSTR2 polynucleotides, or by direct chemical synthesis.

*Protein Purification*

LUSTR2 can be purified from any human cell which expresses the receptor, including those which have been transfected with expression constructs which express LUSTR2. A purified LUSTR2 is separated from other compounds which normally associate with LUSTR2 in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

*Expression of LUSTR2 Polynucleotides*

To express LUSTR2, LUSTR2 polynucleotide can be inserted into an expression vector which contains the necessary elements for the transcription and translation of the inserted coding sequence. Methods which are well known to those skilled in the art can be used to construct expression vectors containing sequences encoding LUSTR2 and appropriate transcriptional and translational control elements. These methods include *in vitro* recombinant DNA techniques, synthetic techniques, and *in vivo* genetic recombination [Devlin et al., Science, (1990)] .

A variety of expression vector/host systems can be utilized to contain and express sequences encoding LUSTR2. These include, but are not limited to, microorganisms, such as bacteria transformed with recombinant bacteriophage, plasmid, or cosmid DNA expression vectors; yeast transformed with yeast expression vectors, insect cell systems infected with virus expression vectors *(e.g.,* baculovirus), plant cell systems transformed with virus expression vectors (*e.g.*, cauliflower mosaic virus, CaMV; tobacco mosaic virus, TMV) or with bacterial expression vectors *(e.g.,* Ti or pBR322 plasmids), or animal cell systems.

The control elements or regulatory sequences are those non-translated regions of the vector - enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (*e.g.,* heat shock, RUBISCO, and storage protein genes) or from plant viruses (*e.g.,* viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding LUSTR2, vectors based on SV40 or EBV can be used with an appropriate selectable marker.

*Bacterial and Yeast Expression Systems*

In bacterial systems, a number of expression vectors can be selected. For example, when a large quantity of LUSTR2 is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding LUSTR2 can be ligated into the vector in frame with sequences for the amino-terminal Met and the subsequent 7 residues of β-galactosidase so that a hybrid protein is produced. pIN vectors or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

*Plant and Insect Expression Systems*

If plant expression vectors are used, the expression of sequences encoding LUSTR2 can be driven by any of a number of promoters. For example, viral promoters such as the 35S and 19S promoters of CaMV can be used alone or in combination with the omega leader sequence from TMV [Scott et al. (1990)]. Alternatively, plant promoters such as the small subunit of RUBISCO or heat shock promoters can be used [Takamatsu et al. (1987)]. These constructs can be introduced into plant cells by direct DNA transformation or by pathogen-mediated transfection.

An insect system also can be used to express LUSTR2. For example, in one such system *Autographa californica* nuclear polyhedrosis virus (AcNPV) is used as a vector to express foreign genes in *Spodoptera frugiperda* cells or in *Trichoplusia* larvae. Sequences encoding LUSTR2 can be cloned into a non-essential region of the virus, such as the polyhedrin gene, and placed under control of the polyhedrin promoter. Successful insertion of LUSTR2 will render the polyhedrin gene inactive and produce recombinant virus lacking coat protein. The recombinant viruses can then be used to infect *S. frugiperda cells* or *Trichoplusia* larvae in which LUSTR2 can be expressed [Fodor et al., (1993)].

*Mammalian Expression Systems*

A number of viral-based expression systems can be used to express LUSTR2 in mammalian host cells. For example, if an adenovirus is used as an expression vector, sequences encoding LUSTR2 can be ligated into an adenovirus transcription/translation complex comprising the late promoter and tripartite leader sequence. Insertion in a non-essential E1 or E3 region of the viral genome can be used to obtain a viable virus which is capable of expressing LUSTR2 in infected host cells [Engelhard et al. (1994)] . If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (*e.g.,* liposomes, polycationic amino polymers, or vesicles). Specific initiation signals also can be used to achieve more efficient translation of sequences encoding LUSTR2. Such signals include the ATG initiation codon and adjacent sequences. In cases where sequences encoding LUSTR2, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic.

*Host Cells*

A host cell strain can be chosen for its ability to modulate the expression of the inserted sequences or to process the expressed LUSTR2 in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g*., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express LUSTR2 can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced LUSTR2 sequences. Resistant clones of stably transformed cells can be proliferated using tissue culture techniques appropriate to the cell type. Any number of selection systems can be used to recover transformed cell lines. These include, but are not limited to, the herpes simplex virus thymidine kinase [Logan & Shenck , (1984)] and adenine phosphoribosyltransferase [Wigler et al., (1977)] genes which can be employed in *tk*- or *aprt*- cells, respectively. Also, antimetabolite, antibiotic, or herbicide resistance can be used as the basis for selection. For example, *dhfr* confers resistance to methotrexate [Lowy et al., (1980)], *npt* confers resistance to the aminoglycosides, neomycin and G-418 [Wigler et al., Proc. Natl. Acad. Sci. (1980)], and *als* and *pat* confer resistance to chlorsulfuron and phosphinotricin acetyltransferase, respectively [Colbere-Garapin et al., (1981)] . Additional selectable genes have been described. For example, *trpB* allows cells to utilize indole in place of tryptophan, or *hisD,* which allows cells to utilize histinol in place of histidine [Murray et al., (1992)]. Visible markers such as anthocyanins, β-glucuronidase and its substrate GUS, and luciferase and its substrate luciferin, can be used to identify transformants and to quantify the amount of transient or stable protein expression attributable to a specific vector system

*Detecting Polypeptide Expression*

Although the presence of marker gene expression suggests that a LUSTR2 polynucleotide is also present, its presence and expression may need to be confirmed. For example, if a sequence encoding LUSTR2 is inserted within a marker gene sequence, transformed cells containing sequences which encode LUSTR2 can be identified by the absence of marker gene function. Alternatively, a marker gene can be placed in tandem with a sequence encoding LUSTR2 under the control of a single promoter. Expression of the marker gene in response to induction or selection usually indicates expression of LUSTR2 polynucleotide.

Alternatively, host cells which contain a LUSTR2 polynucleotide and which express LUSTR2 can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding LUSTR2 can be detected by DNA-DNA or DNA-RNA hybridization or amplification using probes or fragments or fragments of polynucleotides encoding LUSTR2. Nucleic acid amplification-based assays involve the use of oligonucleotides selected from sequences encoding LUSTR2 to detect transformants which contain a LUSTR2 polynucleotide.

A variety of protocols for detecting and measuring the expression of LUSTR2, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on LUSTR2 can be used, or a competitive binding assay can be employed [Hartman & Mulligan, (1988) , Hampton et al. , (1990)] .

A wide variety of labels and conjugation techniques are known by those skilled in the art and can be used in various nucleic acid and amino acid assays. Means for producing labeled hybridization or PCR probes for detecting sequences related to polynucleotides encoding LUSTR2 include oligolabeling, nick translation, end-labeling, or PCR amplification using a labeled nucleotide. Alternatively, sequences encoding LUSTR2 can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding LUSTR2 can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode LUSTR2 can be designed to contain signal sequences which direct secretion of soluble LUSTR2 through a prokaryotic or eukaryotic cell membrane or which direct the membrane insertion of membrane-bound LUSTR2.

As discussed above, other constructions can be used to join a sequence encoding LUSTR2 to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor XA or enterokinase (Invitrogen, San Diego, CA) between the purification domain and LUSTR2 also can be used to facilitate purification. One such expression vector provides for expression of a fusion protein containing LUSTR2 and 6 histidine residues preceding a thioredoxin or an enterokinase cleavage site. The histidine residues facilitate purification by IMAC (immobilized metal ion affinity chromatography) Maddox et al., (1983)], while the enterokinase cleavage site provides a means for purifying LUSTR2 from the fusion protein [Porath et al., (1992)].

*Chemical Synthesis*

Sequences encoding LUSTR2 can be synthesized, in whole or in part, using chemical methods well known in the art [Kroll et al. (1993), Caruthers et al., (1980)].

Alternatively, LUSTR2 itself can be produced using chemical methods to synthesize its amino acid sequence, such as by direct peptide synthesis using solid-phase techniques [Horn et al., (1980); Merrifield et al., (1963)] . Protein synthesis can either be performed using manual techniques or by automation. Automated synthesis can be achieved, for example, using Applied Biosystems 431A Peptide Synthesizer (Perkin Elmer). Optionally, fragments of LUSTR2 can be separately synthesized and combined using chemical methods to produce a full-length molecule.

The newly synthesized peptide can be substantially purified by preparative high performance liquid chromatography. The composition of a synthetic LUSTR2 can be confirmed by amino acid analysis or sequencing. Additionally, any portion of the amino acid sequence of LUSTR2 can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce LUSTR2-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences referred to herein can be engineered using methods generally known in the art to alter LUSTR2-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Antibodies*

Any type of antibody known in the art can be generated to bind specifically to an epitope of LUSTR2. "Antibody" as used herein includes intact immunoglobulin molecules, as well as fragments thereof, such as Fab, $F(ab')_2$, and Fv, which are capable of binding an epitope of LUSTR2. Typically, at least 6, 8, 10, or 12 contiguous amino acids are required to form an epitope. However, epitopes which involve non-contiguous amino acids may require more, e.g., at least 15, 25, or 50 amino acid. An antibody which specifically binds to an epitope of LUSTR2 can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to LUSTR2 provides a detection signal at least 5-, 10-, or 20-fold higher than a detection signal provided with other proteins when used in an immunochemical assay. Preferably, antibodies which specifically bind to LUSTR2 do not detect other proteins in immunochemical assays and can immunoprecipitate LUSTR2 from solution.

LUSTR2 can be used to immunize a mammal, such as a mouse, rat, rabbit, guinea pig, monkey, or human, to produce polyclonal antibodies. If desired, LUSTR2 can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels *(e.g.,* aluminum hydroxide), and surface active substances (*e.g* lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.

Monoclonal antibodies which specifically bind to LUSTR2 can be prepared using any technique which provides for the production of antibody molecules by continuous cell lines in culture. These techniques include, but are not limited to, the hybridoma technique, the human B-cell hybridoma technique, and the EBV-hybridoma technique [Roberge et al., (1995); Kohler et al., (1985); Kozbor et al., (1985); Cote et al., (1983)].

In addition, techniques developed for the production of "chimeric antibodies," the splicing of mouse antibody genes to human antibody genes to obtain a molecule with appropriate antigen specificity and biological activity, can be used [Cole et al. (1984); Morrison et al. (1984); Neuberger et al. (1984)]. Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions.. Antibodies which specifically bind to LUSTR2 can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to LUSTR2. Antibodies with related specificity, but of distinct idiotypic composition, can be generated by chain shuffling from random combinatorial immunoglobin libraries [Takeda et al., (1985)]. Single-chain antibodies also can be constructed using a DNA amplification method, such as PCR, using hybridoma cDNA as a template. Single-chain antibodies can be mono- or bispecific, and can be bivalent or tetravalent. Construction of tetravalent, bispecific single-chain antibodies is taught. A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology [Burton et al. (1991); Verhaar et al. (1995)] .

Antibodies which specifically bind to LUSTR2 also can be produced by inducing *in vivo* production in the lymphocyte population or by screening immunoglobulin libraries or panels of highly specific binding reagents. Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which LUSTR2 is bound. The bound antibodies can then be eluted from the column using a buffer with a high salt concentration.

*Antisense Oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of LUSTR2 gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters.

Modifications of LUSTR2 gene expression can be obtained by designing antisense oligonucleotides which will form duplexes to the control, 5', or regulatory regions of the LUSTR2 gene. Oligonucleotides derived from the transcription initiation site, e.g., between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [Nicholls et al. (1993)]. An antisense oligonucleotide also can be designed to block translation of mRNA by preventing the transcript from binding to ribosomes.

Precise complementarity is not required for successful complex formation between an antisense oligonucleotide and the complementary sequence of a LUSTR2 polynucleotide. Antisense oligonucleotides which comprise, for example, 2, 3, 4, or 5 or more stretches of contiguous nucleotides which are precisely complementary to a LUSTR2 polynucleotide, each separated by a stretch of contiguous nucleotides which are not complementary to adjacent LUSTR2 nucleotides, can provide sufficient targeting specificity for LUSTR2 mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular LUSTR2 polynucleotide sequence. Antisense oligonucleotides can be modified without affecting their ability to hybridize to a LUSTR2 polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art [Gee et al. (1994); Agrawal et al. (1992); Uhlmann et al. (1990)].

*Ribozymes*

Ribozymes are RNA molecules with catalytic activity Uhlmann et al. (1987); Cech et al. (1987), (1990), (1992) ]. Ribozymes can be used to inhibit gene function by cleaving an RNA sequence, as is known in the art (U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences. The coding sequence of a LUSTR2 polynucleotide can be used to generate ribozymes which will specifically bind to mRNA transcribed from a LUSTR2 polynucleotide. Methods of designing and constructing ribozymes which can cleave other RNA molecules in trans in a highly sequence specific manner have been developed and described in the art [Couture & Stinchcomb (1996)]. For example, the cleavage activity of ribozymes can be targeted to specific RNAs by engineering a discrete "hybridization" region into the ribozyme. The hybridization region contains a sequence complementary to the target RNA and thus specifically hybridizes with the Specific ribozyme cleavage sites within a LUSTR2 RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate LUSTR2 RNA targets also can be evaluated by testing accessibility to hybridization with complementary oligonucleotides using ribonuclease protection assays. The nucleotide sequences shown in SEQ ID NO: 1 and its complement provide sources of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease LUSTR2 expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells (U.S. 5,641,673). Ribozymes also can be engineered to provide an additional level of regulation, so that destruction of mRNA occurs only when both a ribozyme and a target gene are induced in the cells.

**Screening / Screening Assays**

*Regulators*

Regulators as used herein, refers to LUSTR2 agonists and LUSTR2 antagonists. Agonists of LUSTR2 are molecules which, when bound to LUSTR2, increase or prolong the activity of LUSTR2. Agonists of LUSTR2 include proteins, nucleic acids, carbohydrates, small molecules, or any other molecule which activate LUSTR2. Antagonists of LUSTR2 are molecules which, when bound to LUSTR2, decrease the amount or the duration of the activity of LUSTR2. Antagonists include proteins, nucleic acids, carbohydrates, antibodies, small molecules, or any other molecule which decrease the activity of LUSTR2.

The term "modulate," as it appears herein, refers to a change in the activity of LUSTR2. For example, modulation may cause an increase or a decrease in protein activity, binding characteristics, or any other biological, functional, or immunological properties of LUSTR2.

As used herein, the terms "specific binding" or "specifically binding" refer to that interaction between a protein or peptide and an agonist, an antibody, or an antagonist. The interaction is dependent upon the presence of a particular structure of the protein recognized by the binding molecule (i.e., the antigenic determinant or epitope). For example, if an antibody is specific for epitope "A," the presence of a polypeptide containing the epitope A, or the presence of free unlabeled A, in a reaction containing free labeled A and the antibody will reduce the amount of labeled A that binds to the antibody.

The invention provides methods (also referred to herein as "screening assays") for identifying compounds which can be used for the treatment of haematological diseases, diseases of the peripheral and central nervous system, chronic obstructive pulmonary disease (COPD), asthma, diseases of the genito-urological system, metabolic diseases and pancreas and heart disorders. The methods entail the identification of candidate or test compounds or agents (e.g., peptides, peptido-mimetics, small molecules or other molecules) which bind to LUSTR2 and/or have a stimulatory or inhibitory effect on the biological activity of LUSTR2 or its expression and then determining which of these compounds have an effect on symtoms or diseases regarding the haematological system, the peripheral and central nervous system, the lung and lung in the state of COPD, asthma, the genito-urological system, metabolic diseases and pancreas and heart disorders in an *in vivo* assay.

Candidate or test compounds or agents which bind to LUSTR2 and/or have a stimulatory or inhibitory effect on the activity or the expression of LUSTR2 are identified either in assays that employ cells which express LUSTR2 on the cell surface (cell-based assays) or in assays with isolated LUSTR2 (cell-free assays). The various assays can employ a variety of variants of LUSTR2 (e.g., full-length LUSTR2, a biologically active fragment of LUSTR2, or a fusion protein which includes all or a portion of LUSTR2). Moreover, LUSTR2 can be derived from any suitable mammalian species (e.g., human LUSTR2, rat LUSTR2 or murine LUSTR2). The assay can be a binding assay entailing direct or indirect measurement of the binding of a test compound or a known LUSTR2 ligand to LUSTR2. The assay can also be an activity assay entailing direct or indirect measurement of the activity of LUSTR2. The assay can also be an expression assay entailing direct or indirect measurement of the expression of LUSTR2 mRNA or LUSTR2 protein. The various screening assays are combined with an *in vivo* assay entailing measuring the effect of the test compound on the symtoms of a haematological, peripheral and central nervous system, COPD, asthma, genito-urological, metabolic diseases and pancreas and heart disorders.

In one embodiment, the invention provides assays for screening candidate or test compounds which bind to or modulate the activity of a membrane-bound (cell surface expressed) form of LUSTR2. Such assays can employ full-length LUSTR2, a biologically active fragment of LUSTR2, or a fusion protein which includes all or a portion of LUSTR2. As described in greater detail below, the test compound can be obtained by any suitable means, e.g., from conventional compound libraries. Determining the ability of the test compound to bind to a membrane-bound form of LUSTR2 can be accomplished, for example, by coupling the test compound with a radioisotope or enzymatic label such that binding of the test compound to the LUSTR2-expressing cell can be measured by detecting the labeled compound in a complex. For example, the test compound can be labeled with ..$^{125}$I, ..$^{35}$S, ..$^{14}$C, or ..$^{3}$H, either directly or indirectly, and the radioisotope detected by direct counting of radioemmission or by scintillation counting. Alternatively, the test compound can be enzymatically labeled with, for example, horseradish peroxidase, alkaline phosphatase, or luciferase, and the enzymatic label detected by determination of conversion of an appropriate substrate to product.

In a competitive binding format, the assay comprises contacting LUSTR2-expressing cell with a known compound which binds to LUSTR2 to form an assay mixture, contacting the assay mixture with a test compound, and determining the ability of the test compound to interact with the LUSTR2-expressing cell, wherein determining the ability of the test compound to interact with the LUSTR2 -expressing cell comprises determining the ability of the test compound to preferentially bind the LUSTR2-expressing cell as compared to the known compound.

In another embodiment, the assay is a cell-based assay comprising contacting a cell expressing a membrane-bound form of LUSTR2 (e.g., full-length LUSTR2, a biologically active fragment of LUSTR2, or a fusion protein which includes all or a portion of LUSTR2) expressed on the cell surface with a test compound and determining the ability of the test compound to modulate (e.g., stimulate or inhibit) the activity of the membrane-bound form of LUSTR2. Determining the ability of the test compound to modulate the activity of the membrane-bound form of LUSTR2 can be accomplished by any method suitable for measuring the activity of LUSTR2, e.g., any method suitable for measuring the activity of a G-protein coupled receptor or other seven-transmembrane receptor (described in greater detail below). The activity of a seven-transmembrane receptor can be measured in a number of ways, not all of which are suitable for any given receptor. Among the measures of activity are: alteration in intracellular Ca.sup.2+ concentration, activation of phospholipase C, alteration in intracellular inositol triphosphate (IP.sub.3) concentration, alteration in intracellular diacylglycerol (DAG) concentration, and alteration in intracellular adenosine cyclic 3', 5'-monophosphate (cAMP) concentration.

Determining the ability of the test compound to modulate the activity of LUSTR2 can be accomplished, for example, by determining the ability of LUSTR2 to bind to or interact with a target molecule. The target molecule can be a molecule with which LUSTR2 binds or interacts with in nature, for example, a molecule on the surface of a cell which expresses LUSTR2, a molecule on the surface of a second cell, a molecule in the extracellular milieu, a molecule associated with the internal surface of a cell membrane or a cytoplasmic molecule. The target molecule can be a component of a signal transduction pathway which facilitates transduction of an extracellular signal (e.g., a signal generated by binding of a LUSTR2 ligand, through the cell membrane and into the cell. The target molecule can be, for example, a second intracellular protein which has catalytic activity or a protein which facilitates the association of downstream signaling molecules with LUSTR2.

Determining the ability of LUSTR2 to bind to or interact with a target molecule can be accomplished by one of the methods described above for determining direct binding. In one embodiment, determining the ability of a polypeptide of the invention to bind to or interact with a target molecule can be accomplished by determining the activity of the target molecule. For example, the activity of the target molecule can be determined by detecting induction of a cellular second messenger of the target (e.g., intracellular Ca.sup.2+, diacylglycerol, IP3, etc.), detecting catalytic/enzymatic activity of the target on an appropriate substrate, detecting the induction of a reporter gene (e.g., a regulatory element that is responsive to a polypeptide of the invention operably linked to a nucleic acid encoding a detectable marker, e.g., luciferase), or detecting a cellular response.

The present invention also includes cell-free assays. Such assays involve contacting a form of LUSTR2 (e.g., full-length LUSTR2, a biologically active fragment of LUSTR2, or a fusion protein comprising all or a portion of LUSTR2) with a test compound and determining the ability of the test compound to bind to LUSTR2. Binding of the test compound to LUSTR2 can be determined either directly or indirectly as described above. In one embodiment, the assay includes contacting LUSTR2 with a known compound which binds LUSTR2 to form an assay mixture, contacting the assay mixture with a test compound, and determining the ability of the test compound to interact with LUSTR2, wherein determining the ability of the test compound to interact with LUSTR2 comprises determining the ability of the test compound to preferentially bind to LUSTR2 as compared to the known compound.

The cell-free assays of the present invention are amenable to use of either a membrane-bound form of LUSTR2 or a soluble fragment thereof. In the case of cell-free assays comprising the membrane-bound form of the polypeptide, it may be desirable to utilize a solubilizing agent such that the membrane-bound form of the polypeptide is maintained in solution. Examples of such solubilizing agents include but are not limited to non-ionic detergents such as n-octylglucoside, n-dodecylglucoside, n-dodecylmaltoside, octanoyl-N-methylglucamide, decanoyl-N-methylglucamide, Triton X-100, Triton X-114, Thesit, Isotridecypoly(ethylene glycol ether)n, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio- 1-propane sulfonate.

In various embodiments of the above assay methods of the present invention, it may be desirable to immobilize LUSTR2 (or a LUSTR2 target molecule) to facilitate separation of complexed from uncomplexed forms of one or both of the proteins, as well as to accommodate automation of the assay. Binding of a test compound to LUSTR2, or interaction of LUSTR2 with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase (GST) fusion proteins or glutathione-S-transferase fusion proteins can be adsorbed onto glutathione sepharose beads (Sigma Chemical; St. Louis, Mo.) or glutathione derivatized microtitre plates, which are then combined with the test compound or the test compound and either the non-adsorbed target protein or LUSTR2, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, arid the level of binding or activity of LUSTR2 can be determined using standard techniques.

Other techniques for immobilizing proteins on matrices can also be used in the screening assays of the invention. For example, either LUSTR2 or its target molecule can be immobilized utilizing conjugation of biotin and streptavidin. Biotinylated polypeptide of the invention or target molecules can be prepared from biotin-NHS (N-hydroxysuccinimide) using techniques well known in the art (e.g., biotinylation kit, Pierce Chemicals; Rockford, Ill.), and immobilized in the wells of streptavidin-coated plates (Pierce Chemical). Alternatively, antibodies reactive with LUSTR2 or target molecules but which do not interfere with binding of the polypeptide of the invention to its target molecule can be derivatized to the wells of the plate, and unbound target or polypeptidede of the invention trapped in the wells by antibody conjugation. Methods for detecting such complexes, in addition to those described above for the GST-immobilized complexes, include immunodetection of complexes using antibodies reactive with LUSTR2 or target molecule, as well as enzyme-linked assays which rely on detecting an enzymatic activity associated with LUSTR2 or target molecule.

The screening assay can also involve monitoring the expression of LUSTR2. For example, regulators of expression of LUSTR2 can be identified in a method in which a cell is contacted with a candidate compound and the expression of LUSTR2 protein or mRNA in the cell is determined. The level of expression of LUSTR2 protein or mRNA the presence of the candidate compound is compared to the level of expression of LUSTR2 protein or mRNA in the absence of the candidate compound. The candidate compound can then be identified as a regulator of expression of LUSTR2 based on this comparison. For example, when expression of LUSTR2 protein or mRNA protein is greater (statistically significantly greater) in the presence of the candidate compound than in its absence, the candidate compound is identified as a stimulator of LUSTR2 protein or mRNA expression. Alternatively, when expression of LUSTR2 protein or mRNA is less (statistically significantly less) in the presence of the candidate compound than in its absence, the candidate compound is identified as an inhibitor of LUSTR2 protein or mRNA expression. The level of LUSTR2 protein or mRNA expression in the cells can be determined by methods described below.

*Binding Assays*

For binding assays, the test compound is preferably a small molecule which binds to and occupies the active site of LUSTR2 receptor polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known LUSTR2 receptor GPCRs and analogues or derivatives thereof.

In binding assays, either the test compound or the LUSTR2 receptor polypeptide can comprise a detectable label, such as a fluorescent, radioisotopic, chemiluminescent, or enzymatic label, such as horseradish peroxidase, alkaline phosphatase, or luciferase. Detection of a test compound which is bound to LUSTR2 receptor polypeptide can then be accomplished, for example, by direct counting of radioemmission, by scintillation counting, or by determining conversion of an appropriate substrate to a detectable product. Alternatively, binding of a test compound to a LUSTR2 receptor polypeptide can be determined without labeling either of the interactants. For example, a microphysiometer can be used to detect binding of a test compound with a LUSTR2 receptor polypeptide. A microphysiometer (*e.g.*, Cytosensor™) is an analytical instrument that measures the rate at which a cell acidifies its environment using a light-addressable potentiometric sensor (LAPS). Changes in this acidification rate can be used as an indicator of the interaction between a test compound and LUSTR2 [Haseloff et al. (1988)].

Determining the ability of a test compound to bind to LUSTR2 also can be accomplished using a technology such as real-time Bimolecular Interaction Analysis (BIA) [McConnell et al. (1992), Sjolander & Urbaniczky (1991)]. BIA is a technology for studying biospecific interactions in real time, without labeling any of the interactants (*e.g.*, BIAcore™). Changes in the optical phenomenon surface plasmon resonance (SPR) can be used as an indication of real-time reactions between biological molecules.

In yet another aspect of the invention, a LUSTR2-like polypeptide can be used as a "bait protein" in a two-hybrid assay or three-hybrid assay [Szabo et al., (1995); Zervos et al. (1993); Madura et al. (1993); Bartel et al. (1993)];

U.S. 5,283,317), to identify other proteins which bind to or interact with LUSTR2 and modulate its activity.

The two-hybrid system is based on the modular nature of most transcription factors, which consist of separable DNA-binding and activation domains. Briefly, the assay utilizes two different DNA constructs. For example, in one construct, polynucleotide encoding LUSTR2 can be fused to a polynucleotide encoding the DNA binding domain of a known transcription factor (*e.g.,* GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e.g.*, LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with LUSTR2.

It may be desirable to immobilize either the LUSTR2 (or polynucleotide) or the test compound to facilitate separation of the bound form from unbound forms of one or both of the interactants, as well as to accommodate automation of the assay. Thus, either the LUSTR2-like polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach LUSTR2-like polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to LUSTR2 (or a polynucleotide encoding for LUSTR2) can be accomplished in any vessel suitable for containing the reactants. Examples of such vessels include microtiter plates, test tubes, and microcentrifuge tubes.

In one embodiment, LUSTR2 is a fusion protein comprising a domain that allows binding of LUSTR2 to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed LUSTR2; the mixture is then incubated under conditions conducive to complex formation (*e.g.*, at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either LUSTR2 (or a polynucleotide encoding LUSTR2) or a test compound can be immobilized utilizing conjugation of biotin and streptavidin. Biotinylated LUSTR2 (or a polynucleotide encoding biotinylated LUSTR2) or test compounds can be prepared from biotin-NHS (N-hydroxysuccinimide) using techniques well known in the art (*e.g.*, biotinylation kit, Pierce Chemicals, Rockford, Ill.) and immobilized in the wells of streptavidin-coated plates (Pierce Chemical). Alternatively, antibodies which specifically bind to LUSTR2, polynucleotide, or a test compound, but which do not interfere with a desired binding site, such as the active site of LUSTR2, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to LUSTR2 receptor polypeptide or test compound, enzyme-linked assays which rely on detecting an activity of LUSTR2 receptor polypeptide, and SDS gel electrophoresis under non-reducing conditions.

Screening for test compounds which bind to a LUSTR2 receptor polypeptide or polynucleotide also can be carried out in an intact cell. Any cell which comprises a LUSTR2 receptor polypeptide or polynucleotide can be used in a cell-based assay system. A LUSTR2 receptor polynucleotide can be naturally occurring in the cell or can be introduced using techniques such as those described above. Binding of the test compound to LUSTR2 or a polynucleotide encoding LUSTR2 is determined as described above.

*Functional Assays*

Test compounds can be tested for the ability to increase or decrease LUSTR2 activity of a LUSTR2 receptor polypeptide. The LUSTR2 activity can be measured, for example, using methods described in the specific examples, below. LUSTR2 activity can be measured after contacting either a purified LUSTR2, a cell membrane preparation, or an intact cell with a test compound. A test compound which decreases LUSTR2 activity by at least about 10, preferably about 50, more preferably about 75, 90, or 100% is identified as a potential agent for decreasing LUSTR2 activity. A test compound which increases LUSTR2 activity by at least about 10, preferably about 50, more preferably about 75, 90, or 100% is identified as a potential agent for increasing LUSTR2 activity.

One such screening procedure involves the use of melanophores which are transfected to express LUSTR2. Such a screening technique is described in PCT WO 92/01810 published Feb. 6, 1992. Thus, for example, such an assay may be employed for screening for a compound which inhibits activation of the receptor polypeptide of the present invention by contacting the melanophore cells which encode the receptor with both the receptor ligand and a compound to be screened. Inhibition of the signal generated by the ligand indicates that a compound is a potential antagonist for the receptor, *i.e.,* inhibits activation of the receptor. The screen may be employed for identifying a compound which activates the receptor by contacting such cells with compounds to be screened and determining whether each compound generates a signal, *i.e.,* activates the receptor.

Other screening techniques include the use of cells which express LUSTR2 (for example, transfected CHO cells) in a system which measures extracellular pH changes caused by receptor activation [Iwabuchi et al. (1993)]. For example, compounds may be contacted with a cell which expresses the receptor polypeptide of the present invention and a second messenger response, *e.g.*, signal transduction or pH changes, can be measured to determine whether the potential compound activates or inhibits the receptor. Another such screening technique involves introducing RNA encoding LUSTR2 into *Xenopus* oocytes to transiently express the receptor. The receptor oocytes can then be contacted with the receptor ligand and a compound to be screened, followed by detection of inhibition or activation of a calcium signal in the case of screening for compounds which are thought to inhibit activation of the receptor.

Another screening technique involves expressing LUSTR2 in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

*Gene Expression*

In another embodiment, test compounds which increase or decrease LUSTR2 gene expression are identified. As used herein, the term "correlates with expression of a "polynucleotide" indicates that the detection of the presence of nucleic acids, the same or related to a nucleic acid sequence encoding LUSTR2, by northern analysis or relatime PCR is indicative of the presence of nucleic acids encoding LUSTR2 in a sample, and thereby correlates with expression of the transcript from the polynucleotide encoding LUSTR2. The term "microarray," as used herein, refers to an array of distinct polynucleotides or oligonucleotides arrayed on a substrate, such as paper, nylon or any other type of membrane, filter, chip, glass slide, or any other suitable solid support. A LUSTR2 polynucleotide is contacted with a test compound, and the expression of an RNA or polypeptide product of LUSTR2 polynucleotide is determined. The level of expression of appropriate mRNA or polypeptide in the presence of the test compound is compared to the level of expression of mRNA or polypeptide in the absence of the test compound. The test compound can then be identified as a regulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of LUSTR2 mRNA or polypeptide expression in the cells can be determined by methods well known in the art for detecting mRNA or polypeptide. Either qualitative or quantitative methods can be used. The presence of polypeptide products of LUSTR2 receptor polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labeled amino acids into LUSTR2.

Such screening can be carried out either in a cell-free assay system or in an intact cell. Any cell which expresses LUSTR2 polynucleotide can be used in a cell-based assay system. The LUSTR2 polynucleotide can be naturally occurring in the cell or can be introduced using techniques such as those described above. Either a primary culture or an established cell line can be used.

*Test Compounds*

Suitable test compounds for use in the screening assays of the invention can be obtained from any suitable source, e.g., conventional compound libraries. The test compounds can also be obtained using any of the numerous approaches in combinatorial library methods known in the art, including: biological libraries; spatially addressable parallel solid phase or solution phase libraries; synthetic library methods requiring deconvolution; the "one-bead one-compound" library method; andsynthetic library methods using affinity chromatography selection. The biological library approach is limited to peptide libraries, while the other four approaches are applicable to peptide, non-peptide oligomer or small molecule libraries of compounds [Lam et al. (1997)].

Examples of methods for the synthesis of molecular libraries can be found in the art [Lam et al. (1997); DeWitt et al. (1993); Erb et al. (1994); Zuckermann et al. (1994); Cho et al. (1993); Carrell et al. (1994) Angew. Chem. Int. Ed. Engl. 33:2059 ]. Libraries of compounds may be presented in solution [Carrell et al. (1994), Angew. Chem. Int. Ed. Engl. 33: 2061; Gallop et al. (1994)] or on beads [ Houghten et al. (1992)], chips [Cull et al. (1992)], bacteria (U.S. 5,223,409), spores (U.S. 5,571,698; 5,403,484; and 5,223,409), plasmids [Coruzzi et al. (1984)] or phage [ Nagarenko et al. (1997); Felici et al. [1991]; Cwirla et al. (1990); Devlin et al. (1990) ; Sambrook et al. (1989)].

*Modeling of Regulators*

Computer modeling and searching technologies permit identification of compounds, or the improvement of already identified compounds, that can modulate LUSTR2 expression or activity. Having identified such a compound or composition, the active sites or regions are identified. Such active sites might typically be ligand binding sites, such as the interaction domain of the ligand with LUSTR2. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

Next, the three dimensional geometric structure of the active site is determined. This can be done by known methods, including X-ray crystallography, which can determine a complete molecular structure. On the other hand, solid or liquid phase NMR can be used to determine certain intramolecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active site, either experimentally, by modeling, or by a combination, candidate modulating compounds can be identified by searching databases containing compounds along with information on their molecular structure. Such a search seeks compounds having structures that match the determined active site structure and that interact with the groups defining the active site. Such a search can be manual, but is preferably computer assisted. These compounds found from this search are potential LUSTR2 modulating compounds.

Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the active site structure of the compound to determine if an improved fit or interaction results. In this manner systematic variations in composition, such as by varying side groups, can be quickly evaluated to obtain modified modulating compounds or ligands of improved specificity or activity.

## Therapeutic Indications and Methods

It was found by the present applicant that LUSTR2 is expressed in different human tissues.

The LUSTR2 receptor is highly expressed in different brain tissues such as cerebellum (total, right, left), parietal lobe, precentral gyrus, thalamus, cerebral cortex, frontal lobe, temporal lobe, postcentral gyrus, tonsilia cerebelli, vermis cerebelli, cerebral penduncles, hippocampus. Other tissues include the heart (atrium, interventricular septum), erythrocytes, trachea, fetal lung, lung, lung in the state of chronic obstructive pulmonary disease (COPD), esophagus, stomach, rectum, cervix, kidney, prostate, prostate in the state of beningn prostate hyperplasia (BPH), salivary gland, thyroid, kidney, pancreas and adipose cells.

The expression in the above mentioned tissues suggests an association between LUSTR2 and peripheral and central nervous system diseases.

*CNS*

Disorders of the nervous system include, but are not limited to brain injuries, cerebrovascular diseases and their consequences, Parkinson's disease, corticobasal degeneration, motor neuron disease, dementia, including ALS, multiple sclerosis, traumatic brain injury, stroke, post-stroke, post-traumatic brain injury, and small-vessel cerebrovascular disease. Dementias, such as Alzheimer's disease, vascular dementia, dementia with Lewy bodies, frontotemporal dementia and Parkinsonism linked to chromosome 17, frontotemporal dementias, including Pick's disease, progressive nuclear palsy, corticobasal degeneration, Huntington's disease, thalamic degeneration, Creutzfeld-Jakob dementia, HIV dementia, schizophrenia with dementia, and Korsakoff's psychosis also can be treated. Similarly, it may be possible to treat cognitive-related disorders, such as mild cognitive impairment, age-associated memory impairment, age-related cognitive decline, vascular cognitive impairment, attention deficit disorders, attention deficit hyperactivity disorders, and memory disturbances in children with learning disabilities, by regulating the activity of LUSTR2.

Pain that is associated with CNS disorders also can be treated by regulating the activity of human LUSTR2. Pain which can be treated includes that associated with central nervous system disorders, such as multiple sclerosis, spinal cord injury, sciatica, failed back surgery syndrome, traumatic brain injury, epilepsy, Parkinson's disease, post-stroke, and vascular lesions in the brain and spinal cord (e.g., infarct, hemorrhage, vascular malformation). Non-central neuropathic pain includes that associated with post mastectomy pain, reflex sympathetic dystrophy (RSD), trigeminal neuralgiaradioculopathy, post-surgical pain, HIV/AIDS related pain, cancer pain, metabolic neuropathies (e.g., diabetic neuropathy, vasculitic neuropathy secondary to connective tissue disease), paraneoplastic polyneuropathy associated, for example, with carcinoma of lung, or leukemia, or lymphoma, or carcinoma of prostate, colon or stomach, trigeminal neuralgia, cranial neuralgias, and post-herpetic neuralgia. Pain associated with peripheral nerve damage, central pain (i.e. due to cerebral ischemia) and various chronic pain i.e., lumbago, back pain (low back pain), inflammatory and/or rheumatic pain.Pain associated with cancer and cancer treatment also can be treated, as can headache pain (for example, migraine with aura, migraine without aura, and other migraine disorders), episodic and chronic tension-type headache, tension-type like headache, cluster headache, and chronic paroxysmal hemicrania.

By regulation of LUSTR2 one can also treat visceral pain as pancreatits, intestinal cystitis, dysmenorrhea, irritable Bowel syndrome, Crohn's disease, biliary colic, ureteral colic, myocardial infarction and pain syndromes of the pelvic cavity, e.g. vulvodynia, orchialgia, urethral syndrome and protatodynia. LUSTR2 can also be used to treat acute pain for example postoperative pain and pain after trauma.

The LUSTR2 receptor can be regulated to treat disorders of the nervous system.

*Hematology*

Hematological disorders include, but are not limited to Acute Myeloic Leukemia, Acute Lymphocytic Leukemia, Chronic Myeloic Leukemia, Chronic Neutrophilic Leukemia, Adult T-cell Leukemia/Lymphoma, Myelodysplastic Syndrome, Multiple Myeloma, Idiopathic thrombocytopenic purpura, Iron deficiency anemia, Megaloblastic anemia (vitamin B12 deficiency), Aplastic Anemia, Thalassemia, Cutaneous T-cell Lymphoma, Malignant Lymphoma bone marrow invasion, Malignant Lymphoma skin invasion, Haemolytic uraemic syndrome, Giant Platelet disease.

The LUSTR2 receptor is highly expressed in erythrocytes and other tissues of the hematological system. The expression in the above mentioned tissues suggests an association between LUSTR2 and hematological diseases.

The LUSTR2 receptor can be regulated in order to treat disorders of the hematological system.

*Cardiovascular diseases*

Heart failure is defined as a pathophysiological state in which an abnormality of cardiac function is responsible for the failure of the heart to pump blood at a rate commensurate with the requirement of the metabolizing tissue. It includes all forms of pumping failures such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina and asymptomatic ischemia.

Arrhythmias include all forms of atrial and ventricular tachyarrhythmias (atrial tachycardia, atrial flutter, atrial fibrillation, atrio-ventricular reentrant tachycardia, preexitation syndrome, ventricular tachycardia, ventricular flutter, ventricular fibrillation) as well as bradycardic forms of arrhythmias.

Hypertensive vascular diseases include primary as well as all kinds of secondary arterial hypertension (renal, endocrine, neurogenic, others). The genes may be used as drug targets for the treatment of hypertension as well as for the prevention of all complications.

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

The LUSTR2 receptor is highly expressed in different tissues of the heart like atrium and the interventricular septum. The expression in the above mentioned tissues suggests an association between LUSTR2 and cardio vascular diseases.

Cardiovascular diseases which can be treated, include but are not limited to the following disorders of the heart and the vascular system: congestive heart failure, myocardial infarction, ischemic diseases of the heart, all kinds of atrial and ventricular arrhythmias, hypertensive vascular diseases and peripheral vascular diseases.

*Genito-urological diseases*

The receptor is highly expressed in different genito-urological tissues as cervix, kidney, prostate and in prostate in the state of benigne prostate hyperplasia (BPH). The expression of LUSTR2 in BPH is 5 times higher than in a healthy prostate. The expression in the above mentioned tissues suggests an association between LUSTR2 and genito-urological diseases.

The LUSTR2 receptor can be regulated to treat disorders of the genito-urological system. Genito-urological disorders which may be treated include, but not limited to benigne prostatic hyperplasia (BHP) and urinary incontinence.

*Asthma / COPD diseases*

Allergy is a complex process in which environmental antigens induce clinically adverse reactions. Asthma can be understood as an basically allergic disease of the lung and its tissues. The asthma inducing antigens, called allergens, typically elicit a specific IgE response and, although in most cases the allergens themselves have little or no intrinsic toxicity, they induce pathology when the IgE response in turn elicits an IgE-dependent or T cell-dependent hypersensitivity reaction. Hypersensitivity reactions can be local or systemic and typically occur within minutes after allergen exposure in individuals who have previously been sensitized to the respective allergen. The hypersensitivity reaction of allergy develops when the allergen is recognized by IgE antibodies bound to specific receptors on the surface of effector cells, such as mast cells, basophils, or eosinophils, which causes the activation of the effector cells and the release of mediators that produce the acute signs and symptoms of the reactions. Allergic diseases include asthma, allergic rhinitis (hay fever), atopic dermatitis, and anaphylaxis.

Asthma is though to arise as a result of interactions between multiple genetic and environmental factors and is characterized by three major features: 1) intermittent and reversible airway obstruction caused by bronchoconstriction, increased mucus production, and thickening of the walls of the airways that leads to a narrowing of the airways, 2) airway hyperresponsiveness, and 3) airway inflammation. Certain cells are critical to the inflammatory reaction of asthma and they include T cells and antigen presenting cells, B cells that produce IgE, and mast cells, basophils, eosinophils, and other cells that bind IgE. These effector cells accumulate at the site of allergic reaction in the airways and release toxic products that contribute to the acute pathology and eventually to tissue destruction related to the disorder. Other resident cells, such as smooth muscle cells, lung epithelial cells, mucus-producing cells, and nerve cells may also be abnormal in individuals with asthma and may contribute to its pathology. While the airway obstruction of asthma, presenting clinically as an intermittent wheeze and shortness of breath, is generally the most pressing symptom of the disease requiring immediate treatment, the inflammation and tissue destruction associated with the disease can lead to irreversible changes that eventually makes asthma a chronic and disabling disorder requiring long-term management.

Despite recent important advances in our understanding of the pathophysiology of allergies and asthma, they appear to be increasing in prevalence and severity [Cawkwell et al. (1993)] . It is estimated that 30-40% of the population suffer with atopic allergy, and 15% of children and 5% of adults in the population suffer from asthma. Thus, an enormous burden is placed on our health care resources. However, both diagnosis and treatment of asthma are difficult. The severity of lung tissue inflammation is not easy to measure and the symptoms of the disease are often indistinguishable from those of respiratory infections, chronic respiratory inflammatory disorders, allergic rhinitis, or other respiratory disorders. Often, the inciting allergen cannot be determined, making removal of the causative environmental agent difficult. Current pharmacological treatments suffer their own set of disadvantages. Commonly used therapeutic agents, such as beta agonists, can act as symptom relievers to transiently improve pulmonary function, but do not affect the underlying inflammation. Agents that can reduce the underlying inflammation, such as antiinflammatory steroids, may have major drawbacks which range from immunosuppression to bone loss. In addition, many of the present therapies, such as inhaled corticosteroids, are short-lasting, inconvenient to use, and must be used often on a regular, in some cases lifelong basis, making failure of patients to comply with the treatment a major problem and thereby reducing their effectiveness as a treatment. Because of the problems associated with conventional therapies, alternative treatment strategies have been evaluated. Glycophorin A, cyclosporin and a nonapeptide fragment of IL-2 all inhibit interleukin-2 dependent T lymphocyte proliferation; however, they are known to have many other effects. For example, cyclosporin is used as a immunosuppressant after organ transplantation. While these agents may represent alternatives to steroids in the treatment of asthmatics, they inhibit interleukin-2 dependent T lymphocyte proliferation and potentially critical immune functions associated with homeostasis. Other treatments that block the release or activity of mediators of bronchoconstriction, such as cromones or anti-leukotrienes, have recently been introduced for the treatment of mild asthma, but they are expensive and not effective in all patients and it is unclear whether they affect the chronic changes associated with asthmatic inflammation at all. What is needed in the art is the identification of a treatment that can act on pathways critical to the development of asthma and that both blocks the episodic attacks of the disorder and which dampens the hyperactive allergic immune response without immuno-compromising the patient.

Chronic obstructive pulmonary (or airways) disease (COPD) is a condition defined physiologically as airflow obstruction that generally results from a mixture of emphysema and peripheral airway obstruction due to chronic bronchitis [ Botstein et al. (1980)]. Emphysema is characterised by destruction of alveolar walls leading to abnormal enlargement of the air spaces of the lung. Chronic bronchitis is defined clinically as the presence of chronic productive cough for three months in each of two successive years. In COPD, airflow obstruction is usually progressive and is only partially reversible. By far the most important risk factor for development of COPD is cigarette smoking, although the disease does also occur in non-smokers.

Chronic inflammation of the airways is a key pathological feature of COPD. The inflammatory cell population comprises increased numbers of macrophages, neutrophils and $CD8^+$ lymphocyes. Inhaled irritants such as cigarette smoke activate macrophages resident in the respiratory tract as well as epithelial cells leading to release of chemokines (e.g. interleukin-8) and other chemotactic factors which act to increase the neutrophil/monocyte trafficking from the blood into lung tissue and airways. Neutrophils and monocytes recruited into the airways can release a variety of potentially damaging mediators such as proteolytic enzymes and reactive oxygen species. Matrix degradation and emphysema, along with airway wall thickening, surfactant dysfunction and mucus hypersecretion are all potential sequelae of this inflammatory response that lead to impaired airflow and gas exchange.

The LUSTR2 receptor is highly expressed in different respiratory tissues such as trachea, lung, fetal lung, lung in the state of Chronic Obstructive Pulmonary Disease (COPD) and asthma and the expression of LUSTR2 in COPD affected lung is higher than in healthy lung. The expression of LUSTR2 in the above mentioned tissues suggests an association between LUSTR2 and respiratory diseases such as asthma and COPD, and therapeutic regulation of the LUSTR2 receptor can be used to treat disorders of the respiratory system.

*Diseases of the pancreas*

Disorders of the pancreas include, but are not limited to pearson -marrow pancreas syndrome, nesidioblastosis of pancreas, shwachman-diamond syndrome, asplenia, cystic fibrosis, pancreatic hypoplasia, diabetes, pancreatic carcinoma, von hippel-lindau syndrome, johanson-blizzard syndrome, polycystic kidney disease, multiple endocrine neoplasia, polycystic kidney and hepatic disease, kearns-sayre syndrome. The LUSTR2 receptor is highly expressed in tissues of the pancreas. The expression of LUSTR2 in healthy pancreas is 8 times higher than in pancreas e.g. from liver cirrhosis patients. The expression in the above mentioned tissues suggests an association between LUSTR2 and pancreas disorders , and therapeutic regulation of the LUSTR2 receptor can be used to treat disorders of the pancreas.

*Metabolic disease*

Obesity and overweight are defined as an excess of body fat relative to lean body mass. An increase in caloric intake or a decrease in energy expenditure or both can bring about this imbalance leading to surplus energy being stored as fat. Obesity is associated with important medical morbidities and an increase in mortality. The causes of obesity are poorly understood and may be due to genetic factors, environmental factors or their combination, to cause a positive energy balance. In contrast, anorexia and cachexia are characterized by an imbalance in energy intake versus energy expenditure leading to a negative energy balance and weight loss. Agents that either increase energy expenditure and/or decrease energy intake, absorption or storage would be useful for treating obesity, overweight, and associated comorbidities. Agents that either increase energy intake and/or decrease energy expenditure or increase the amount of lean tissue would be useful for treating cachexia, anorexia and wasting disorders.

This gene, translated proteins and agents which modulate this gene or portions of the gene or its products are useful for treating obesity, overweight, anorexia, cachexia, wasting disorders, appetite suppression, appetite enhancement, increases or decreases in satiety, modulation of body weight, and/or other eating disorders such as bulimia. Also this gene, translated proteins and agents which modulate this gene or portions of the gene or its products are useful for treating obesity/overweight-associated comorbidities including hypertension; type 2 diabetes; coronary artery disease; hyperlipidemia; stroke; gallbladder disease; gout; osteoarthritis; sleep apnea and respiratory problems; some types of cancer including endometrial, breast, prostate and colon; thrombotic disease; polycystic ovarian syndrome; reduced fertility; complications of pregnancy; menstrual irregularities; hirsutism; stress incontinence and depression.

The LUSTR2 receptor is highly expressed in adipose cells. Its expression in adipose tissue suggests an association between LUSTR2 and metabolic diseases. The LUSTR2 receptor can be regulated to treat disorders of the adipose tissue and adipositas.

*Applications*

The present invention provides for both prophylactic and therapeutic methods for haemaotological, peripheral and central nervous system, COPD, asthma, genito-urological, metabolic diseases, pancreas and heart disorders.

The regulatory method of the invention involves contacting a cell with an agent that modulates one or more of the activities of LUSTR2. An agent that modulates activity can be an agent as described herein, such as a nucleic acid or a protein, a naturally-occurring cognate ligand of the polypeptide, a peptide, a peptidomimetic, or any small molecule. In one embodiment, the agent stimulates one or more of the biological activities of LUSTR2. Examples of such stimulatory agents include the active LUSTR2 and nucleic acid molecules encoding a portion of LUSTR2. In another embodiment, the agent inhibits one or more of the biological activities of LUSTR2. Examples of such inhibitory agents include antisense nucleic acid molecules and antibodies. These regulatory methods can be performed in vitro (e.g., by culturing the cell with the agent) or, alternatively, in vivo (e.g, by administering the agent to a subject). As such, the present invention provides methods of treating an individual afflicted with a disease or disorder characterized by unwanted expression or activity of LUSTR2 or a protein in the LUSTR2 signaling pathway. In one embodiment, the method involves administering an agent like any agent identified or being identifiable by a screening assay as described herein, or combination of such agents that modulate say upregulate or downregulate the expression or activity of LUSTR2 or of any protein in the LUSTR2 signaling pathway. In another embodiment, the method involves administering a regulator of LUSTR2 as therapy to compensate for reduced or undesirably low expression or activity of LUSTR2 or a protein in the LUSTR2 signalling pathway.

Stimulation of activity or expression of LUSTR2 is desirable in situations in which activity or expression is abnormally low and in which increased activity is likely to have a beneficial effect. Conversely, inhibition of activity or expression of LUSTR2 is desirable in situations in which activity or expression of LUSTR2 is abnormally high and in which decreasing its activity is likely to have a beneficial effect.

This invention is further illustrated by the following examples which should not be construed as limiting. The contents of all references, patents and published patent applications cited throughout this application are hereby incorporated by reference.

**Pharmaceutical Compositions**

This invention further pertains to novel agents identified by the above-described screening assays and uses thereof for treatments as described herein.

The nucleic acid molecules, polypeptides, and antibodies (also referred to herein as "active compounds") of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The invention includes pharmaceutical compositions comprising a regulator of LUSTR2 expression or activity (and/or a regulator of the activity or expression of a protein in the LUSTR2 signalling pathway) as well as methods for preparing such compositions by combining one or more such regulators and a pharmaceutically acceptable carrier. Also within the invention are pharmaceutical compositions comprising a regulator identified using the screening assays of the invention packaged with instructions for use. For regulators that are antagonists of LUSTR2 activity or which reduce LUSTR2 expression, the instructions would specify use of the pharmaceutical composition for treatment of haematological, peripheral and central nervous system, COPD, asthma, genito-urological, metabolic diseases and pancreas and heart disorders. For regulators that are agonists of LUSTR2 activity or increase LUSTR2 expression, the instructions would specify use of the pharmaceutical composition for treatment of haemaotological, peripheral and central nervous system, COPD, asthma, genito-urological, metabolic diseases and pancreas and heart disorders.

An antagonist of LUSTR2 may be produced using methods which are generally known in the art. In particular, purified LUSTR2 may be used to produce antibodies or to screen libraries of pharmaceutical agents to identify those which specifically bind LUSTR2. Antibodies to LUSTR2 may also be generated using methods that are well known in the art. Such antibodies may include, but are not limited to, polyclonal, monoclonal, chimeric, single chain antibodies, Fab fragments, and fragments produced by a Fab expression library. Neutralizing antibodies like those which inhibit dimer formation are especially preferred for therapeutic use.

In another embodiment of the invention, the polynucleotides encoding LUSTR2, or any fragment or complement thereof, may be used for therapeutic purposes. In one aspect, the complement of the polynucleotide encoding LUSTR2 may be used in situations in which it would be desirable to block the transcription of the mRNA. In particular, cells may be transformed with sequences complementary to polynucleotides encoding LUSTR2. Thus, complementary molecules or fragments may be used to modulate LUSTR2 activity, or to achieve regulation of gene function. Such technology is now well known in the art, and sense or antisense oligonucleotides or larger fragments can be designed from various locations along the coding or control regions of sequences encoding LUSTR2.

Expression vectors derived from retroviruses, adenoviruses, or herpes or vaccinia viruses, or from various bacterial plasmids, may be used for delivery of nucleotide sequences to the targeted organ, tissue, or cell population. Methods which are well known to those skilled in the art can be used to construct vectors which will express nucleic acid sequence complementary to the polynucleotides of the gene encoding LUSTR2. checklit: These techniques are described, for example, in [Scott and Smith (1990) Science 249:386-390].

Any of the therapeutic methods described above may be applied to any subject in need of such therapy, including, for example, mammals such as dogs, cats, cows, horses, rabbits, monkeys, and most preferably, humans.

An additional embodiment of the invention relates to the administration of a pharmaceutical composition containing LUSTR2 in conjunction with a pharmaceutically acceptable carrier, for any of the therapeutic effects discussed above. Such pharmaceutical compositions may consist of LUSTR2, antibodies to LUSTR2, and mimetics, agonists, antagonists, or inhibitors of LUSTR2. The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs or hormones.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EL.TM. (BASF; Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin. Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration. For pharmaceutical compositions which include an antagonist of LUSTR2 activity, a compound which reduces expression of LUSTR2, or a compound which reduces expression or activity of a protein in the LUSTR2 signaling pathway or any combination thereof, the instructions for administration will specify use of the composition for haematological, peripheral and central nervous system, COPD, asthma, genito-urological, metabolic diseases and pancreas and heart disorders. For pharmaceutical compositions which include an agonist of LUSTR2 activity, a compound which increases expression of LUSTR2, or a compound which increases expression or activity of a protein in the LUSTR2 signaling pathway or any combination thereof, the instructions for administration will specify use of the composition for haematological, peripheral and central nervous system, COPD, asthma, genito-urological, metabolic diseases and pancreas and heart disorders.

## Diagnostics

In another embodiment, antibodies which specifically bind LUSTR2 may be used for the diagnosis of disorders characterized by the expression of LUSTR2, or in assays to monitor patients being treated with LUSTR2 or agonists, antagonists, and inhibitors of LUSTR2. Antibodies useful for diagnostic purposes may be prepared in the same manner as those described above for therapeutics. Diagnostic assays for LUSTR2 include methods which utilize the antibody and a label to detect LUSTR2 in human body fluids or in extracts of cells or tissues. The antibodies may be used with or without modification, and may be labeled by covalent or non-covalent joining with a reporter molecule. A wide variety of reporter molecules, several of which are described above, are known in the art and may be used.

A variety of protocols for measuring LUSTR2, including ELISAs, RIAs, and FACS, are known in the art and provide a basis for diagnosing altered or abnormal levels of LUSTR2 expression. Normal or standard values for LUSTR2 expression are established by combining body fluids or cell extracts taken from normal mammalian subjects, preferably human, with antibody to LUSTR2 under conditions suitable for complex formation The amount of standard complex formation may be quantified by various methods, preferably by photometric means. Quantities of LUSTR2 expressed in subject samples from biopsied tissues are compared with the standard values. Deviation between standard and subject values establishes the parameters for diagnosing disease.

In another embodiment of the invention, the polynucleotides encoding LUSTR2 may be used for diagnostic purposes. The polynucleotides which may be used include oligonucleotide sequences, complementary RNA and DNA molecules, and PNAs. The polynucleotides may be used to detect and quantitate gene expression in biopsied tissues in which expression of LUSTR2 may be correlated with disease. The diagnostic assay may be used to distinguish between absence, presence, and excess expression of LUSTR2, and to monitor regulation of LUSTR2 levels during therapeutic intervention.

Polynucleotide sequences encoding LUSTR2 may be used for the diagnosis of a haemaotological, peripheral and central nervous system, COPD, asthma, genito-urological, metabolic diseases and pancreas and heart disorders disorder associated with expression of LUSTR2. The polynucleotide sequences encoding LUSTR2 may be used in Southern- , Northern-, or dot-blot analysis, or other membrane-based technologies; in PCR technologies; in dipstick, pin, and ELISA assays; and in microarrays utilizing fluids or tissues from patient biopsies to detect altered LUSTR2 expression. Such qualitative or quantitative methods are well known in the art.

In a particular aspect, the nucleotide sequences encoding LUSTR2 may be useful in assays that detect the presence of associated disorders, particularly those mentioned above. The nucleotide sequences encoding LUSTR2 may be labeled by standard methods and added to a fluid or tissue sample from a patient under conditions suitable for the formation of hybridization complexes. After a suitable incubation period, the sample is washed and the signal is quantitated and compared with a standard value. If the amount of signal in the patient sample is significantly altered from that of a comparable control sample, the nucleotide sequences have hybridized with nucleotide sequences in the sample, and the presence of altered levels of nucleotide sequences encoding LUSTR2 in the sample indicates the presence of the associated disorder. Such assays may also be used to evaluate the efficacy of a particular therapeutic treatment regimen in animal studies, in clinical trials, or in monitoring the treatment of an individual patient.

In order to provide a basis for the diagnosis of haemaotological, peripheral and central nervous system, COPD, asthma, genito-urological, metabolic diseases and pancreas and heart disorders associated with expression of LUSTR2, a normal or standard profile for expression is established. This may be accomplished by combining body fluids or cell extracts taken from normal subjects, either animal or human, with a sequence, or a fragment thereof, encoding LUSTR2, under conditions suitable for hybridization or amplification. Standard hybridization may be quantified by comparing the values obtained from normal subjects with values from an experiment in which a known amount of a substantially purified polynucleotide is used. Standard values obtained from normal samples may be compared with values obtained from samples from patients who are symptomatic for a disorder. Deviation from standard values is used to establish the presence of a disorder.

Another technique for drug screening which may be used provides for high throughput screening of compounds having suitable binding affinity to the protein of interest as described in published PCT application WO84/03564. In this method, large numbers of different small test compounds are synthesized on a solid substrate, such as plastic pins or some other surface. The test compounds are reacted with LUSTR2, or fragments thereof, and washed. Bound LUSTR2 is then detected by methods well known in the art. Purified LUSTR2 can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable of binding LUSTR2 specifically compete with a testcompound for binding LUSTR2. In this manner, antibodies can be used to detect the presence of any peptide which shares one or more antigenic determinants with LUSTR2.

G-protein coupled receptors are ubiquitous in the mammalian host and are responsible for many biological functions, including many pathologies. Accordingly, it is desirable to find compounds and drugs which stimulate a G-protein coupled receptor on the one hand and which can inhibit the function of a G-protein coupled receptor on the other hand. For example, compounds which activate the G-protein coupled receptor may be employed for therapeutic purposes, such as the treatment of: asthma, Parkinson's disease, acute heart failure, urinary retention, and osteoporosis. In particular, compounds which activate the receptors of the present invention are useful in treating various cardiovascular ailments such as caused by the lack of pulmonary blood flow or hypertension. In addition these compounds may also be used in treating various physiological disorders relating to abnormal control of fluid and electrolyte homeostasis and in diseases associated with abnormal angiotensininduced aldosterone secretion.

In general, compounds which inhibit activation of the G-protein coupled receptor may be employed for a variety of therapeutic purposes, for example, for the treatment of hypotension and/or hypertension, angina pectoris, myocardial infarction, ulcers, asthma, allergies, benign prostatic hypertrophy, and psychotic and neurological disorders including schizophrenia, manic excitement, depression, delirium, dementia or severe mental retardation, dyskinesias, such as Huntington's disease or Tourett's syndrome, among others. Compounds which inhibit G-protein coupled receptors have also been useful in reversing endogenous anorexia and in the control of bulimia.

**Determination of a Therapeutically Effective Dose**

The determination of a therapeutically effective dose is well within the capability of those skilled in the art. A therapeutically effective dose refers to that amount of active ingredient which increases or decreases LUSTR2 activity relative to LUSTR2 activity which occurs in the absence of the therapeutically effective dose. For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration. The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 micrograms to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun," and DEAE- or calcium phosphate-mediated transfection.

If the expression product is mRNA, the reagent is preferably an antisense oligonucleotide or a ribozyme. Polynucleotides which express antisense oligonucleotides or ribozymes can be introduced into cells by a variety of methods, as described above. Preferably, a reagent reduces expression of LUSTR2 gene or the activity of LUSTR2 by at least about 10, preferably about 50, more preferably about 75, 90, or 100% relative to the absence of the reagent. The effectiveness of the mechanism chosen to decrease the level of expression of LUSTR2 gene or the activity of LUSTR2 can be assessed using methods well known in the art, such as hybridization of nucleotide probes to LUSTR2-specific mRNA, quantitative RT-PCR, immunologic detection of LUSTR2, or measurement of LUSTR2 activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects. Any of the therapeutic methods described above can be applied to any subject in need of such therapy, including, for example, mammals such as dogs, cats, cows, horses, rabbits, monkeys, and most preferably, humans.

Nucleic acid molecules of the invention are those nucleic acid molecules which are contained in a group of nucleic acid molecules consisting of (i) nucleic acid molecules encoding a polypeptide comprising the amino acid sequence of SEQ ID NO:2, (ii) nucleic acid molecules comprising the sequence of SEQ ID NO:1, (iii) nucleic acid molecules having the sequence of SEQ ID NO:1, (iv)nucleic acid molecules the complementary strand of which hybridizes under stringent conditions to a nucleic acid molecule of (i), (ii), or (iii); and (v) nucleic acid molecules the sequence of which differs from the sequence of a nucleic acid molecule of (iii) due to the degeneracy of the genetic code, wherein the polypeptide encoded by said nucleic acid molecule has LUSTR2 activity.

Polypeptides of the invention are those polypeptides which are contained in a group of polypeptides consisting of (i)polypeptides having the sequence of SEQ ID NO:2,

(ii) polypeptides comprising the sequence of SEQ ID NO:2, (iii) polypeptides encoded by nucleic acid molecules of the invention and (iv) polypeptides which show at least 99%, 98%, 95%, 90%, or 80% homology with a polypeptide of (i), (ii), or
(iii), wherein said purified polypeptide has LUSTR2 activity.

It is an objective of the invention to provide a vector comprising the nucleic acid molecule of the invention.
Another object of the invention is a host cell containing a vector of the invention.
Another object of the invention is a method of producing a LUSTR2 comprising the steps of (i) culturing a host cell of the invention under suitable conditions and (ii) recovering the LUSTR2 from the culture medium.
Another object of the invention is a method for the detection of a polynucleotide encoding a LUSTR2 in a sample comprising the steps of (i) hybridizing a polynucleotide of the invention to nucleic acid material of the sample, thereby forming a hybridization complex; and (ii) detecting said hybridization complex.
Another object of the invention is a method for the detection of a polynucleotide encoding a LUSTR2 in a sample comprising the steps of (i) hybridizing a polynucleotide of the invention to nucleic acid material of the sample, thereby forming a hybridization complex; and (ii) detecting said hybridization complex, wherein, before hybridization, the nucleic acid material of the sample is amplified.
Another object of the invention is a method for the detection of a polynucleotide of the invention or a polypeptide of the invention comprising the steps of (i) contacting a sample with a reagent which specifically interacts with a polynucleotide of the invention or a polypeptide of the invention, and (ii) detecting said interaction.
Another object of the invention are diagnostic kits for conducting any of the methods above.
Regulators of a given protein, within the meaning of the invention, are understood as being compounds which alter either directly or indirectly the activity of the given protein either in vivo or in vitro. Alteration of the activity can be, e.g. , but not limited to, by allosteric effects or by affecting the expression of the given protein.
Other objects of the invention are methods for screening for regulators of the activity of a LUSTR2 comprising the steps of (i) contacting a test compound with a polypeptide of the invention, (ii) detect binding of said test compound to said polypeptide of the invention, wherein test compounds that bind under (ii) are identified as potential regulators of the LUSTR2 activity.
Other objects of the invention are methods of the above, wherein the step of contacting is in or at the surface of a cell.
Other objects of the invention are methods of the above, wherein the step of contacting is in or at the surface of a cell wherein the cell is in vitro.
Other objects of the invention are methods of the above, wherein the step of contacting is in a cell-free system.
Other objects of the invention are methods of the above, wherein the polypeptide of the invention is coupled to a detectable label.
Other objects of the invention are methods of the above, wherein the compound is coupled to a detectable label.
Other objects of the invention are methods of the above, wherein the test compound displaces a ligand which is first bound to the polypeptide.
Other objects of the invention are methods of the above, wherein the polypeptide of the invention is attached to a solid support.
Other objects of the invention are methods of the above, wherein the compound is attached a solid support.
Another object of the invention is a method of screening for regulators of the activity of a LUSTR2 comprising the steps of

(i) measuring the activity of a polypeptide of the invention at a certain concentration of a test compound or in the absence of said test compound, (ii) measuring the activity of said polypeptide at a different concentrations of said test compound, wherein said test compound is identified as a regulator of the activity of a LUSTR2 when there is a significant difference between the activities measured in (i) and (ii). Another object of the invention is a method of screening for regulators of the activity of a LUSTR2 comprising the steps of (i) measuring the activity of a polypeptide of the invention at a certain concentration of a test compound, (ii) measuring the activity of a polypeptide of the invention at the presence of a compound known to be a regulator of LUSTR2.

Another object of the invention is a method of screening for regulators of the activity of a LUSTR2 comprising the aforementioned methods, wherein the activities are measured in a cell.

Another object of the invention is a method of screening for regulators of the activity of a LUSTR2 comprising the aforementioned methods, wherein the cell is in vitro.

Another object of the invention is a method of screening for regulators of the activity of a LUSTR2 comprising the aforementioned methods, wherein the activities are measured in a cell-free system.

Another object of the invention is a method of screening for regulators of LUSTR2 comprising the steps of (i) contacting a test compound with a nucleic acid molecule of the invention, (ii) detect binding of said test compound to said nucleic acid molecule, wherein said test compound is identified as a potential regulator of LUSTR2 when it binds to said nucleic acid molecule.

Another object of the invention is a method of screening for regulators of LUSTR2 comprising the steps of (i) contacting a test compound with a nucleic acid molecule of the invention, wherein the nucleic acid molecule is an RNA (ii) detect binding of said test compound to said RNA molecule, wherein said test compound is identified as a potential regulator of LUSTR2 when it binds to said RNA molecule.

Another object of the invention is a method of screening for regulators of LUSTR2 comprising the steps of contacting a test compound with a nucleic acid molecule of the invention, detect binding of said test compound to said nucleic acid molecule, wherein said test compound is identified as a potential regulator of LUSTR2 when it binds to said nucleic acid molecule, wherein the contacting step is (i) in or at the surface of a cell or (ii) in a cell-free system or wherein (iii) the polypeptide or nucleic acid molecule is coupled to a detectable label or wherein (iv) the test compound is coupled to a detectable label.

Another object of the invention is a method of regulating the activity of a LUSTR2 wherein LUSTR2 is contacted with a regulator of LUSTR2.

Another object of the invention is a method of diagnosing a LUSTR2 related disease in a diseased mammal comprising the steps of (i) measuring the amount of a nucleic acid molecule of the invention in a sample taken from said diseased mammal, (ii) comparing the result of (i) to the amount of said nucleic acid molecule in one or several healthy mammals, wherein a LUSTR2 related disease is diagnosed in the diseased mammal when the amount of said nucleic acid molecule in the diseased mammal is significantly different from the amount of said nucleic acid molecule in the healthy mammal/mammals.

Other objects of the invention are pharmaceutical compositions comprising (i) a nucleic acid molecule of the invention, (ii) a vector of the invention, or (iii) a polypeptide of the invention.

Another object of the invention are pharmaceutical compositions comprising a regulator of the invention.

Another object of the invention are pharmaceutical compositions comprising a regulator identified by methods of the invention for the treatment of hematological disorders, peripheral and central nervous system disorders, COPD, asthma, genito-urological disorders, metabolic diseases, pancreas disorders or heart disorders in a mammal.

Another object of the invention regards the use of regulators of a LUSTR2 as identified by any of the aforementioned methods for the preparation of pharmaceutical compositions useful for the treatment of hematological disorders, peripheral and central nervous system disorders, COPD, asthma, genito-urological disorders, metabolic diseases, pancreas disorders or heart disorders in a mammal.

Another object of the invention are methods for the preparation of pharmaceutical compositions useful for the treatment of hematological disorders, peripheral and central nervous system disorders, COPD, asthma, genito-urological disorders, metabolic diseases, pancreas disorders or heart disorders in a mammal comprising the steps of (i) identifying a regulator of LUSTR2 by any of the aforementioned methods, (ii) determining of whether said regulator ameliorates the symptoms of hematological disorders, peripheral and central nervous system disorders, COPD, asthma, genito-urological disorders, metabolic diseases, pancreas disorders or heart disorders in a mammal, (iii) combining of said regulator with an acceptable pharmaceutical carrier.

Another object of the invention is the use of a regulator of LUSTR2 as identified by any of the aforementioned methods for (i) the treatment of hematological disorders, peripheral and central nervous system disorders, COPD, asthma, genito-urological disorders, metabolic diseases, pancreas disorders or heart disorders in a mammal, or (ii) use of a regulator of LUSTR2 for the regulation of LUSTR2 activity in a mammal having a hematological disorder, peripheral and central nervous system disorder, COPD, asthma, a genito-urological disorder, a metabolic disease, a pancreas disorder or a heart disorder.

Another object of the invention is the use of any of the aforementioned pharmaceutical compositions wherein the regulator of LUSTR2 is either a small molecule, an RNA molecule, or an antisense oligonucleotide, or a polypeptide, an antibody, or a ribozyme. Small molecules, within the meaning of the invention, are organic molecules of a molecular weight of less than one thousand five hundred grams per mol.

The examples below are provided to illustrate the subject invention. These examples are provided by way of illustration and are not included for the purpose of limiting the invention.

## Examples

### Example 1: Search for homologous sequences in public sequence data bases

The degree of homology can readily be calculated by known methods. Preferred methods to determine homology are designed to give the largest match between the sequences tested. Methods to determine homology are codified in publicly available computer programs such as BestFit, BLASTP, BLASTN, and FASTA. The BLAST programs are publicly available from NCBI and other sources in the internet.

For LUSTR2 the following hits to known sequences were identified by using the BLAST algorithm [Altschul et al. (1997)] and the following set of parameters: matrix = BLOSUM62 and low complexity filter. The following databases were searched: NCBI (non-redundant database) and DERWENT patent database (Geneseq). The following hits were found:

>trembl|AX073578|AX073578_1 unnamed ORF; Sequence 30 from Patent W00104297. Length = 543 Score = 1096 bits (2803), Expect = 0.0, Identities = 543/543 (100%), Positives = 543/543 (100%), Frame = +1

>trembl|AL365404|IR1703145_1 product: "hypothetical protein, similar to (AAF46469.1) CG12, Length = 536, Score = 1083 bits (2771), Expect = 0.0, Identities = 536/536 (100%), Positives = 536/536 (100%), Frame = +1

>tremblnew|AF376726|AF376726_1 gene: "LUSTR2"; product: "lung seven transmembrane receptor, Length = 562, Score = 902 bits (2305), Expect = 0.0, Identities = 453/564 (80%), Positives = 490/564 (86%), Gaps = 21/564 (3%), Frame = +1

>tremblnew|AF376725|AF376725_1 gene: "LUSTR1"; product: "lung seven transmembrane receptor, Length = 552, Score = 521 bits (1328), Expect = e-147, Identities = 282/545 (51%), Positives = 363/545 (65%), Gaps = 17/545 (3%), Frame = +1

>trembl|AB046844|AB046844_1 gene: "KIAA1624"; product: "KIAA1624 protein"; Homo sapiens, Length = 599, Score = 498 bits (1269), Expect = e-140, Identities = 282/593 (47%), Positives = 363/593 (60%), Gaps = 65/593 (10%), Frame = +1

>trembl|AK019012|AK019012_1 unnamed ORF; Mus musculus 10 day old male pancreas cDNA, RIK, Length = 220, Score = 427 bits (1086), Expect = e-118, Identities = 207/220 (94%), Positives = 216/220 (98%), Frame = +1

Hence, the amino acid sequence of SEQ ID NO:2 is not previously known to code for a LUSTR2 polypeptide.

### Example 2: Expression profiling

Total cellular RNA was isolated from cells by one of two standard methods: 1)guanidine isothiocyanate/Cesium chloride density gradient centrifugation [Kellogg et al. (1990)]; or with the Tri-Reagent protocol according to the manufacturer's specifications (Molecular Research Center, Inc., Cincinatti, Ohio). Total RNA prepared by the Tri-reagent protocol was treated with DNAse I to remove genomic DNA contamination.

For relative quantitation of the mRNA distribution of LUSTR2, total RNA from each cell or tissue source was first reverse transcribed. 85 μg of total RNA was reverse transcribed using 1 μmole random hexamer primers, 0.5 mM each of dATP, dCTP, dGTP and dTTP (Qiagen, Hilden, Germany), 3000 U RnaseQut (Invitrogen, Groningen, Netherlands) in a final volume of 680 μl. The first strand synthesis buffer and Omniscript reverse transcriptase (2 u/μl) were from (Qiagen, Hilden, Germany). The reaction was incubated at 37°C for 90 minutes and cooled on ice. The volume was adjusted to 6800 μl with water, yielding a final concentration of 12.5 ng/μl of starting RNA.

For relative quantitation of the distribution of LUSTR2 mRNA in cells and tissues the Perkin Elmer ABI Prism RTM. 7700 Sequence Detection system or Biorad iCycler was used according to the manufacturer's specifications and protocols. PCR reactions were set up to quantitate LUSTR2 and the housekeeping genes HPRT (hypoxanthine phosphoribosyltransferase), GAPDH (glyceraldehyde-3-phosphate dehydrogenase), β-actin, and others. Forward and reverse primers and probes for LUSTR2 were designed using the Perkin Elmer ABI Primer Express™ software and were synthesized by TibMolBiol (Berlin, Germany). The LUSTR2 forward primer sequence was: Primed (SEQ ID NO:3). The LUSTR2 reverse primer sequence was Primer2 (SEQ ID NO:5). Probe1 (SEQ ID NO:4), labelled with FAM (carboxyfluorescein succinimidyl ester) as the reporter dye and TAMRA (carboxytetra-methylrhodamine) as the quencher, is used as a probe for LUSTR2. The following reagents were prepared in a total of 25 μl : 1x TaqMan buffer A, 5.5 mM $MgCl_2$, 200 nM of dATP, dCTP, dGTP, and dUTP, 0.025 U/μl AmpliTaq Gold™, 0.01 U/μl AmpErase and Probe1 (SEQ ID NO: 4), LUSTR2 forward and reverse primers each at 200 nM, 200 nM LUSTR2 FAM/TAMRA-labelled probe, and 5 μl of template cDNA. Thermal cycling parameters were 2 min at 50°C, followed by 10 min at 95°C, followed by 40 cycles of melting at 95°C for 15 sec and annealing/extending at 60°C for 1 min.

*Calculation of corrected CT values*

The CT (threshold cycle) value is calculated as described in the "Quantitative determination of nucleic acids" section. The CF-value (factor for threshold cycle correction) is calculated as follows :

1. PCR reactions were set up to quantitate the housekeeping genes (HKG) for each cDNA sample.

2. $CT_{HKG}$-values (threshold cycle for housekeeping gene) were calculated as described in the "Quantitative determination of nucleic acids" section.

3. $CT_{HKG}$-mean values (CT mean value of all HKG tested on one cDNAs) of all HKG for each cDNA are calculated (n = number of HKG):

$$CT_{HKG\text{-}n}\text{-mean value} = (CT_{HKG1}\text{-value} + CT_{HKG2}\text{-value} + \ldots + CT_{HKG\text{-}n}\text{-value}) /$$

$$n$$

4. $CT_{pannel}$ mean value (CT mean value of all HKG in all tested cDNAs) =

$$(CT_{HKG1}\text{-mean value} + CT_{HKG2}\text{-mean value} + \ldots + CT_{HKG\text{-}y}\text{-mean value}) / y$$

(y = number of cDNAs)

5. $CF_{cDNA\text{-}n}$ (correction factor for cDNA n) = $CT_{pannel}$-mean value - $CT_{HKG\text{-}n}$ - mean value

6. $CT_{cDNA\text{-}n}$ (CT value of the tested gene for the cDNA n) + $CF_{cDNA\text{-}n}$ (correction factor for cDNA n) = $CT_{cor\text{-}cDNA\text{-}n}$ (corrected CT value for a gene on cDNA n)

*Calculation of relative expression*

Definition : highest $CT_{cor\text{-}cDNA\text{-}n} \neq 40$ is defined as $CT_{cor\text{-}cDNA}$ [high]

$$\text{Relative Expression} = 2^{(CTcor\text{-}cDNA[high]\ -\ CTcor\text{-}CDNA\text{-}n)}$$

*Human tissues*

Coronary smooth muscle cells, brain, testis, pancreas, stomach, cerebellum, trachea, adrenal gland, skeletal muscle, salivary gland, small intestine, prostata, fetal liver, placenta, fetal brain, uterus, mammary gland, heart, spleen, lung, HeLa cells, liver, kidney, thymus, bone marrow, thyroid, colon, bladder, spinal cord, peripheral blood, liver liver cirrhosis, pancreas liver cirrhosis, spleen liver cirrhosis, total Alzheimer brain, fetal lung, breast tumor, colon tumor, lung tumor, HEK 293 cells, adipose, pericardium, fetal heart, thyroid tumor, MDA MB 231 cells, HEP G2 cells, HUVEC cells, fetal kidney, breast, Jurkat T-cells, Alzheimer brain cortex, cervix, esophagus, thalamus, precentral gyrus, hippocampus, occipital lobe, cerebral penduncles, postcentral gyms, temporal lobe, parietal lobe, cerebellum (right), cerebellum (left), tonsilla cerebelli, cerebral meninges, pons, frontal lobe, cerebral cortex, corpus callosum, vermis cerebelli, Alzheimer brain frontal lobe, interventricular septum, heart atrium (right), heart atrium (left), heart ventricle (left), skin, ileum chronic inflammation, erythrocyte, aorta, lung in the state of COPD, aorta sclerotic, prostate in the state of BPH, rectum, retina, penis, penis corpus cavernosum, artery, vein, lymphnode, dorsal root ganglia, thrombocytes.

*Expression profile*

The results of the the mRNA-quantification (expression profiling) is shown in Table 1.

Table 1:

| Relative expression of LUSTR2 in various human tissues. | |
|---|---|
| **Tissue** | **relative expression** |
| Brain | 22,0 |
| total Alzheimer brain | 15,6 |
| fetal brain | 21,0 |
| Cerebral cortex | 54,3 |
| Alzheimer brain cortex | 48,0 |
| frontal lobe | 14,0 |
| Alzheimer brain frontal lobe | 50,2 |
| Cerebellum | 35,1 |
| Cerebellum (right) | 290,1 |
| Cerebellum (left) | 168,9 |
| Tonsilla cerebelli | 97,6 |
| Precentral gyrus | 149,4 |
| Hippocampus | 70,5 |
| Occipital lobe | 9,1 |
| Cerebral penduncles | 67,3 |
| Postcentral gyrus | 35,2 |
| Temporal lobe | 79,6 |
| Parietal lobe | 268,4 |
| Cerebral meninges | 24,4 |
| Pons | 69,9 |
| Corpus callosum | 10,3 |
| Vermis cerebelli | 41,5 |
| spinal cord | 24,8 |
| Thalamus | 1229,3 |
| dorsal root ganglia | 5,4 |
| Retina | 8,0 |
| Heart | 5,6 |
| fetal heart | 11,2 |
| Heart, Interventricular septum | 80,3 |
| heart atrium (right) | 269,6 |
| heart atrium (left) | 44,5 |
| heart ventricle (left) | 7,5 |
| Heart, Pericardium | 21,4 |
| Peripheral blood | 12,1 |
| Erythrocyte | 77,4 |
| Aorta | 341,2 |
| aorta sclerotic | 46,5 |
| Artery | 183,3 |
| Vein | 6,2 |
| Coronary smooth muscle cells | 30,1 |
| HUVEC cells | 10,5 |
| Thyroid | 45,9 |
| Thyroid tumor | 30,5 |
| Spleen | 12,5 |
| Spleen liver cirrhosis | 49,9 |
| Thymus | 27,2 |
| bone marrow | 7,2 |

Table 1: (continued)

| Relative expression of LUSTR2 in various human tissues. | |
|---|---|
| **Tissue** | **relative expression** |
| Lymphnode | 3,0 |
| Jurkat T-cells | 2,6 |
| Thrombocytes | 1,2 |
| | |
| Lung | 158,3 |
| fetal lung | 38,1 |
| lung tumor | 15,7 |
| lung COPD | 62,9 |
| | |
| Liver | 28,6 |
| fetal liver | 6,2 |
| liver liver cirrhosis | 8,3 |
| HEP G2 cells | 49,7 |
| Pancreas | 597,8 |
| Pancreas liver cirrhosis | 60,1 |
| Stomach | 55,7 |
| small intestine | 14,3 |
| Colon | 9,0 |
| colon tumor | 11,0 |
| Rectum | 21,2 |
| ileum chronic inflammation | 240,0 |
| Esophagus | 53,7 |
| | |
| Kidney | 98,7 |
| fetal kidney | 34,1 |
| HEK 293 cells | 15,0 |
| Skeletal muscle | 4,1 |
| Cervix | 28,5 |
| HeLa cells | 15,3 |
| Breast | 61,6 |
| breast tumor | 61,0 |
| Mammary gland | 62,8 |
| MDA MB 231 cells | 7,7 |
| Testis | 76,7 |
| Penis | 6,1 |
| Corpus cavernosum | 1,0 |
| Trachea | 98,8 |
| Adrenal gland | 16,5 |
| Salivary gland | 53,6 |
| Skin | 27,0 |
| Bladder | 16,4 |
| Prostata | 57,3 |
| Prostate BPH | 50,3 |
| Placenta | 31,2 |
| Uterus | 28,7 |
| Adipose | 147,2 |

**Example 3: Antisense Analysis**

Knowledge of the correct, complete cDNA sequence coding for LUSTR2 enables its use as a tool for antisense technology in the investigation of gene function. Oligonucleotides, cDNA or genomic fragments comprising the antisense strand of a polynucleotide coding for LUSTR2 are used either in vitro or in vivo to inhibit translation of the mRNA. Such technology is now well known in the art, and antisense molecules can be designed at various locations along the nucleotide sequences. By treatment of cells or whole test animals with such antisense sequences, the gene of interest is effectively turned off. Frequently, the function of the gene is ascertained by observing behavior at the intracellular, cellular, tissue or organismal level (e.g., lethality, loss of differentiated function, changes in morphology, etc.).
In addition to using sequences constructed to interrupt transcription of a particular open reading frame, modifications of gene expression is obtained by designing antisense sequences to intron regions, promoter/enhancer elements, or even to transacting regulatory genes.

**Example 4: Expression of LUSTR2**

Expression of LUSTR2 is accomplished by subcloning the cDNAs into appropriate expression vectors and transfecting the vectors into expression hosts such as, e.g., *E. coli.* In a particular case, the vector is engineered such that it contains a promoter for β-galactosidase, upstream of the cloning site, followed by sequence containing the amino-terminal Methionine and the subsequent seven residues of β-galactosidase. Immediately following these eight residues is an engineered bacteriophage promoter useful for artificial priming and transcription and for providing a number of unique endonuclease restriction sites for cloning.
Induction of the isolated, transfected bacterial strain with Isopropyl-β-D-thiogalacto-pyranoside (IPTG) using standard methods produces a fusion protein corresponding to the first seven residues of β-galactosidase, about 15 residues of "linker", and the peptide encoded within the cDNA. Since cDNA clone inserts are generated by an essentially random process, there is probability of 33% that the included cDNA will lie in the correct reading frame for proper translation. If the cDNA is not in the proper reading frame, it is obtained by deletion or insertion of the appropriate number of bases using well known methods including in vitro mutagenesis, digestion with exonuclease III or mung bean nuclease, or the inclusion of an oligonucleotide linker of appropriate length.
The LUSTR2 cDNA is shuttled into other vectors known to be useful for expression of proteins in specific hosts. Oligonucleotide primers containing cloning sites as well as a segment of DNA (about 25 bases) sufficient to hybridize to stretches at both ends of the target cDNA is synthesized chemically by standard methods. These primers are then used to amplify the desired gene segment by PCR. The resulting gene segment is digested with appropriate restriction enzymes under standard conditions and isolated by gel electrophoresis. Alternately, similar gene segments are produced by digestion of the cDNA with appropriate restriction enzymes. Using appropriate primers, segments of coding sequence from more than one gene are ligated together and cloned in appropriate vectors. It is possible to optimize expression by construction of such chimeric sequences.
Suitable expression hosts for such chimeric molecules include, but are not limited to, mammalian cells such as Chinese Hamster Ovary (CHO) and human 293 cells., insect cells such as Sf9 cells, yeast cells such as *Saccharomyces cerevisiae* and bacterial cells such as *E. coli.* For each of these cell systems, a useful expression vector also includes an origin of replication to allow propagation in bacteria, and a selectable marker such as the β-lactamase antibiotic resistance gene to allow plasmid selection in bacteria. In addition, the vector may include a second selectable marker such as the neomycin phosphotransferase gene to allow selection in transfected eukaryotic host cells. Vectors for use in eukaryotic expression hosts require RNA processing elements such as 3' polyadenylation sequences if such are not part of the cDNA of interest.
Additionally, the vector contains promoters or enhancers which increase gene expression. Such promoters are host specific and include MMTV, SV40, and metallothionine promoters for CHO cells; trp, lac, tac and T7 promoters for bacterial hosts; and alpha factor, alcohol oxidase and PGH promoters for yeast. Transcription enhancers, such as the rous sarcoma virus enhancer, are used in mammalian host cells. Once homogeneous cultures of recombinant cells are obtained through standard culture methods, large quantities of recombinantly produced LUSTR2 are recovered from the conditioned medium and analyzed using chromatographic methods known in the art. For example, LUSTR2 can be cloned into the expression vector pcDNA3, as exemplified herein. This product can be used to transform, for example, HEK293 or COS by methodology standard in the art. Specifically, for example, using Lipofectamine (Gibco BRL catolog no. 18324-020) mediated gene transfer.

**Example 5: Isolation of Recombinant LUSTR2**

LUSTR2 is expressed as a chimeric protein with one or more additional polypeptide domains added to facilitate protein purification. Such purification facilitating domains include, but are not limited to, metal chelating peptides such as histidine-tryptophan modules that allow purification on immobilized metals [Appa Rao (1997)] and the domain utilized in the FLAGS extension/affinity purification system (Immunex Corp., Seattle, Washington). The inclusion of a cleavable linker sequence such as Factor Xa or enterokinase (Invitrogen, Groningen, The Netherlands) between the purification domain and the LUSTR2 sequence is useful to facilitate expression of LUSTR2.

**Example 6: Testing of Chimeric GPCRs**

Functional chimeric GPCRs are constructed by combining the extracellular receptive sequences of a new isoform with the transmembrane and intracellular segments of a known isoform for test purposes. This concept was demonstrated by Kobilka et al. (1988), Science 240:1310-1316) who created a series of chimeric α2-β2 adrenergic receptors (AR) by inserting progressively greater amounts of α2-AR transmembrane sequence into β2-AR. The binding activity of known agonists changed as the molecule shifted from having more α2 than β2 conformation, and intermediate constructs demonstrated mixed specificity. The specificity for binding antagonists, however, correlated with the source of the domain VII. The importance of T7G domain VII for ligand recognition was also found in chimeras utilizing two yeast α-factor receptors and is significant because the yeast receptors are classified as miscellaneous receptors. Thus, functional role of specific domains appears to be preserved throughout the GPCR family regardless of category.

In parallel fashion, internal segments or cytoplasmic domains from a particular isoform are exchanged with the analogous domains of a known GPCRs and used to identify the structural determinants responsible for coupling the receptors to trimeric G-proteins. A chimeric receptor in which domains V, VI, and the intracellular connecting loop from β2-AR were substituted into α2-AR was shown to bind ligands with α2-AR specificity, but to stimulate adenylate cyclase in the manner of β2-AR. This demonstrates that for adrenergic-type receptors, G-protein recognition is present in domains V and VI and their connecting loop. The opposite situation was predicted and observed for a chimera in which the V->VI loop from α1-AR replaced the corresponding domain on β2-AR and the resulting receptor bound ligands with β2-AR specificity and activated G-protein-mediated phosphatidylinositol turnover in the α1-AR manner. Finally, chimeras constructed from muscarinic receptors also demonstrated that V->VI loop is the major determinant for specificity of G-protein activity.

Chimeric or modified GPCRs containing substitutions in the extracellular and transmembrane regions have shown that these portions of the receptor determine ligand binding specificity. For example, two Serine residues conserved in domain V of all adrenergic and D catecholainine GPCRs are necessary for potent agonist activity. These serines are believed to form hydrogen bonds with the catechol moiety of the agonists within the GPCR binding site. Similarly, an Asp residue present in domain III of all GPCRs which bind biogenic amines is believed to form an ion pair with the ligand amine group in the GPCR binding site.

Functional, cloned GPCRs are expressed in heterologous expression systems and their biological activity assessed. One heterologous system introduces genes for a mammalian GPCR and a mammalian G-protein into yeast cells. The GPCR is shown to have appropriate ligand specificity and affinity and trigger appropriate biological activation (growth arrest and morphological changes) of the yeast cells.

An alternate procedure for testing chimeric receptors is based on the procedure utilizing the purinergic receptor ($P_2u$). Function is easily tested in cultured K562 human leukemia cells because these cells lack $P_2u$ receptors. K562 cells are transfected with expression vectors containing either normal or chimeric $P_2u$ and loaded with fura-a, fluorescent probe for $Ca^{++}$. Activation of properly assembled and functional $P_2u$ receptors with extracellular UTP or ATP mobilizes intracellular $Ca^{++}$ which reacts with fura-a and is measured spectrofluorometrically.

As with the GPCRs above, chimeric genes are created by combining sequences for extracellular receptive segments of any new GPCR polypeptide with the nucleotides for the transmembrane and intracellular segments of the known $P_2u$ molecule. Bathing the transfected K562 cells in microwells containing appropriate ligands triggers binding and fluorescent activity defining effectors of the GPCR molecule. Once ligand and function are established, the $P_2u$ system is useful for defining antagonists or inhibitors which block binding and prevent such fluorescent reactions.

**Example 7: Production of LUSTR2 Specific Antibodies**

Two approaches are utilized to raise antibodies to LUSTR2, and each approach is useful for generating either polyclonal or monoclonal antibodies. In one approach, denatured protein from reverse phase HPLC separation is obtained in quantities up to 75 mg. This denatured protein is used to immunize mice or rabbits using standard protocols; about 100 µg are adequate for immunization of a mouse, while up to 1 mg might be used to immunize a rabbit. For identifying mouse hybridomas, the denatured protein is radioiodinated and used to screen potential murine B-cell hybridomas for those which produce antibody. This procedure requires only small quantities of protein, such that 20 mg is sufficient for labeling and screening of several thousand clones.

In the second approach, the amino acid sequence of an appropriate LUSTR2 domain, as deduced from translation of the cDNA, is analyzed to determine regions of high antigenicity. Oligopeptides comprising appropriate hydrophilic regions are synthesized and used in suitable immunization protocols to raise antibodies. The optimal amino acid sequences for immunization are usually at the C-terminus, the N-terminus and those intervening, hydrophilic regions of the polypeptide which are likely to be exposed to the external environment when the protein is in its natural conformation.

Typically, selected peptides, about 15 residues in length, are synthesized using an Applied Biosystems Peptide Synthesizer Model 431A using fmoc-chemistry and coupled to keyhole limpet hemocyanin (KLH; Sigma, St. Louis, MO) by reaction with M-maleimidobenzoyl-N-hydroxysuccinimide ester, MBS. If necessary, a cysteine is introduced at the N-terminus of the peptide to permit coupling to KLH. Rabbits are immunized with the peptide-KLH complex in complete Freund's adjuvant. The resulting antisera are tested for antipeptide activity by binding the peptide to plastic, blocking with 1% bovine serum albumin, reacting with antisera, washing and reacting with labeled (radioactive or fluorescent), affinity purified, specific goat anti-rabbit IgG.

Hybridomas are prepared and screened using standard techniques. Hybridomas of interest are detected by screening with labeled LUSTR2 to identify those fusions producing the monoclonal antibody with the desired specificity. In a typical protocol, wells of plates (FAST; Becton-Dickinson, Palo Alto, CA) are coated during incubation with affinity purified, specific rabbit anti-mouse (or suitable antispecies 1 g) antibodies at 10 mg/ml. The coated wells are blocked with 1% bovine serum albumin, (BSA), washed and incubated with supernatants from hybridomas. After washing the wells are incubated with labeled LUSTR2 at 1 mg/ml. Supernatants with specific antibodies bind more labeled LUSTR2 than is detectable in the background. Then clones producing specific antibodies are expanded and subjected to two cycles of cloning at limiting dilution. Cloned hybridomas are injected into pristane-treated mice to produce ascites, and monoclonal antibody is purified from mouse ascitic fluid by affinity chromatography on Protein A. Monoclonal antibodies with affinities of at least

$10^8$ $M^{-1}$, preferably $10^9$ to $10^{10}$ $M^{-1}$ or stronger, are typically made by standard procedures.

**Example 8: Diagnostic Test Using LUSTR2 Specific Antibodies**

Particular LUSTR2 antibodies are useful for investigating signal transduction and the diagnosis of infectious or hereditary conditions which are characterized by differences in the amount or distribution of LUSTR2 or downstream products of an active signaling cascade.

Diagnostic tests for LUSTR2 include methods utilizing antibody and a label to detect LUSTR2 in human body fluids, membranes, cells, tissues or extracts of such. The polypeptides and antibodies of the present invention are used with or without modification. Frequently, the polypeptides and antibodies are labeled by joining them, either covalently or noncovalently, with a substance which provides for a detectable signal. A wide variety of labels and conjugation techniques are known and have been reported extensively in both the scientific and patent literature. Suitable labels include radionuclides, enzymes, substrates, cofactors, inhibitors, fluorescent agents, chemiluminescent agents, chromogenic agents, magnetic particles and the like.

A variety of protocols for measuring soluble or membrane-bound LUSTR2, using either polyclonal or monoclonal antibodies specific for the protein, are known in the art. Examples include enzyme-linked immunosorbent assay (ELISA), radioimmunoassay (RIA) and fluorescent activated cell sorting (FACS). A two-site monoclonal-based immunoassay utilizing monoclonal antibodies reactive to two non-interfering epitopes on LUSTR2 is preferred, but a competitive binding assay may be employed.

**Example 9: Purification of Native LUSTR2 Using Specific Antibodies**

Native or recombinant LUSTR2 is purified by immunoaffinity chromatography using antibodies specific for LUSTR2. In general, an immunoaffinity column is constructed by covalently coupling the anti-TRH antibody to an activated chromatographic resin. Polyclonal immunoglobulins are prepared from immune sera either by precipitation with ammonium sulfate or by purification on immobilized Protein A (Pharmacia LKB Biotechnology, Piscataway N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated Sepharose (Pharmacia LKB Biotechnology). The antibody is coupled to the resin, the resin is blocked, and the derivative resin is washed according to the manufacturer's instructions.

Such immunoaffinity columns are utilized in the purification of LUSTR2 by preparing a fraction from cells containing LUSTR2 in a soluble form. This preparation is derived by solubilization of whole cells or of a subcellular fraction obtained via differential centrifugation (with or without addition of detergent) or by other methods well known in the art. Alternatively, soluble LUSTR2 containing a signal sequence is secreted in useful quantity into the medium in which the cells are grown.

A soluble LUSTR2-containing preparation is passed over the immunoaffinity column, and the column is washed under conditions that allow the preferential absorbance of LUSTR2 (e.g., high ionic strength buffers in the presence of detergent). Then, the column is eluted under conditions that disrupt antibody/protein binding (e.g., a buffer of pH 2-3 or a high concentration of a chaotrope such as urea or thiocyanate ion), and LUSTR2 is collected.

**Example 10: Drug Screening**

This invention is particularly useful for screening therapeutic compounds by using LUSTR2 or binding fragments thereof in any of a variety of drug screening techniques. As LUSTR2 is a G protein coupled receptor any of the methods commonly used in the art may potentially be used to identify LUSTR2 ligands. For example, the activity of a G protein coupled receptor such as LUSTR2 can be measured using any of a variety of appropriate functional assays in which activation of the receptor results in an observable change in the level of some second messenger system, such as adenylate cyclase, guanylylcyclase, calcium mobilization, or inositol phospholipid hydrolysis. Alternatively, the polypeptide or fragment employed in such a test is either free in solution, affixed to a solid support, borne on a cell surface or located intracellularly. One method of drug screening utilizes eukaryotic or prokaryotic host cells which are stably transformed with recombinant nucleic acids expressing the polypeptide or fragment. Drugs are screened against such transformed cells in competitive binding assays. Such cells, either in viable or fixed form, are used for standard binding assays.

Measured, for example, is the formation of complexes between LUSTR2 and the agent being tested. Alternatively, one examines the diminution in complex formation between LUSTR2 and a ligand caused by the agent being tested.

Thus, the present invention provides methods of screening for drug canditates, drugs, or any other agents which affect signal transduction. These methods, well known in the art, comprise contacting such an agent with LUSTR2 polypeptide or a fragment thereof and assaying (i) for the presence of a complex between the agent and LUSTR2 polypeptide or fragment, or (ii) for the presence of a complex between LUSTR2 polypeptide or fragment and the cell. In such competitive binding assays, the LUSTR2 polypeptide or fragment is typically labeled. After suitable incubation, free LUSTR2 polypeptide or fragment is separated from that present in bound form, and the amount of free or uncomplexed label is a measure of the ability of the particular agent to bind to LUSTR2 or to interfere with the LUSTR2-agent complex.

Another technique for drug screening provides high throughput screening for compounds having suitable binding affinity to LUSTR2 polypeptides. Briefly stated, large numbers of different small peptide test compounds are synthesized on a solid substrate, such as plastic pins or some other surface. The peptide test compounds are reacted with LUSTR2 polypeptide and washed. Bound LUSTR2 polypeptide is then detected by methods well known in the art. Purified LUSTR2 are also coated directly onto plates for use in the aforementioned drug screening techniques. In addition, non-neutralizing antibodies are used to capture the peptide and immobilize it on the solid support.

This invention also contemplates the use of competitive drug screening assays in which neutralizing antibodies capable of binding LUSTR2 specifically compete with a test compound for binding to LUSTR2 polypeptides or fragments thereof. In this manner, the antibodies are used to detect the presence of any peptide which shares one or more antigenic determinants with LUSTR2.

**Example 11: Rational Drug Design**

The goal of rational drug design is to produce structural analogs of biologically active polypeptides of interest or of small molecules with which they interact, agonists, antagonists, or inhibitors. Any of these examples are used to fashion drugs which are more active or stable forms of the polypeptide or which enhance or interfere with the function of a polypeptide in vivo.

In one approach, the three-dimensional structure of a protein of interest, or of a protein-inhibitor complex, is determined by x-ray crystallography, by computer modeling or, most typically, by a combination of the two approaches. Both the shape and charges of the polypeptide must be ascertained to elucidate the structure and to determine active site(s) of the molecule. Less often, useful information regarding the structure of a polypeptide is gained by modeling based on the structure of homologous proteins. In both cases, relevant structural information is used to design efficient inhibitors. Useful examples of rational drug design include molecules which have improved activity or stability or which act as inhibitors, agonists, or antagonists of native peptides.

It is also possible to isolate a target-specific antibody, selected by functional assay, as described above, and then to solve its crystal structure. This approach, in principle, yields a pharmacore upon which subsequent drug design is based. It is possible to bypass protein crystallography altogether by generating anti-idiotypic antibodies (anti-ids) to a functional, pharmacologically active antibody. As a mirror image of a mirror image, the binding site of the anti-ids is expected to be an analog of the original receptor. The anti-id is then used to identify and isolate peptides from banks of chemically or biologically produced peptides. The isolated peptides then act as the pharmacore.

By virtue of the present invention, sufficient amount of polypeptide are made available to perform such analytical studies as X-ray crystallography. In addition, knowledge of the LUSTR2 amino acid sequence provided herein provides guidance to those employing computer modeling techniques in place of or in addition to x-ray crystallography.

**Example 12: Identification of Other Members of the Signal Transduction Complex**

The inventive purified LUSTR2 is a research tool for identification, characterization and purification of interacting G or other signal transduction pathway proteins. Radioactive labels are incorporated into a selected LUSTR2 domain by various methods known in the art and used in vitro to capture interacting molecules. A preferred method involves labeling the primary amino groups in LUSTR2 with $^{125}$I Bolton-Hunter reagent. This reagent has been used to label various molecules without concomitant loss of biological activity.

Labeled LUSTR2 is useful as a reagent for the purification of molecules with which it interacts. In one embodiment of affinity purification, membrane-bound LUSTR2 is covalently coupled to a chromatography column. Cell-free extract derived from synovial cells or putative target cells is passed over the column, and molecules with appropriate affinity bind to LUSTR2. LUSTR2-complex is recovered from the column, and the LUSTR2-binding ligand disassociated and subjected to N-terminal protein sequencing. The amino acid sequence information is then used to identify the captured molecule or to design degenerate oligonucleotide probes for cloning the relevant gene from an appropriate cDNA library.

In an alternate method, antibodies are raised against LUSTR2, specifically monoclonal antibodies. The monoclonal antibodies are screened to identify those which inhibit the binding of labeled LUSTR2. These monoclonal antibodies are then used therapeutically.

**Example 13: Use and Administration of Antibodies, Inhibitors, or Antagonists**

Antibodies, inhibitors, or antagonists of LUSTR2 or other treatments and compunds that are limiters of signal transduction (LSTs), provide different effects when administered therapeutically. LSTs are formulated in a nontoxic, inert, pharmaceutically acceptable aqueous carrier medium preferably at a pH of about 5 to 8, more preferably 6 to 8, although pH may vary according to the characteristics of the antibody, inhibitor, or antagonist being formulated and the condition to be treated. Characteristics of LSTs include solubility of the molecule, its half-life and antigenicity/immunogenicity. These and other characteristics aid in defining an effective carrier. Native human proteins are preferred as LSTs, but organic or synthetic molecules resulting from drug screens are equally effective in particular situations.

LSTs are delivered by known routes of administration including but not limited to topical creams and gels; transmucosal spray and aerosol; transdermal patch and bandage; injectable, intravenous and lavage formulations; and orally administered liquids and pills particularly formulated to resist stomach acid and enzymes. The particular formulation, exact dosage, and route of administration is determined by the attending physician and varies according to each specific situation.

Such determinations are made by considering multiple variables such as the condition to be treated, the LST to be administered, and the pharmacokinetic profile of a particular LST. Additional factors which are taken into account include severity of the disease state, patient's age, weight, gender and diet, time and frequency of LST administration, possible combination with other drugs, reaction sensitivities, and tolerance/response to therapy. Long acting LST formulations might be administered every 3 to 4 days, every week, or once every two weeks depending on half-life and clearance rate of the particular LST.

Normal dosage amounts vary from 0.1 to $10^5$ μg, up to a total dose of about 1 g, depending upon the route of administration. Guidance as to particular dosages and methods of delivery is provided in the literature; see U.S. Pat. Nos. 4,657,760; 5,206,344; or 5,225,212. Those skilled in the art employ different formulations for different LSTs. Administration to cells such as nerve cells necessitates delivery in a manner different from that to other cells such as vascular endothelial cells.

It is contemplated that abnormal signal transduction, trauma, or diseases which trigger LUSTR2 activity are treatable with LSTs. These conditions or diseases are specifically diagnosed by the tests discussed above, and such testing should be performed in suspected cases of viral, bacterial or fungal infections, allergic responses, mechanical injury associated with trauma, hereditary diseases, lymphoma or carcinoma, or other conditions which activate the genes of lymphoid or neuronal tissues.

**Example 14: Production of Non-human Transgenic Animal**s

Animal model systems which elucidate the physiological and behavioral roles of the LUSTR2 receptor are produced by creating nonhuman transgenic animals in which the activity of the LUSTR2 receptor is either increased or decreased, or the amino acid sequence of the expressed LUSTR2 receptor is altered, by a variety of techniques. Examples of these techniques include, but are not limited to: 1) Insertion of normal or mutant versions of DNA encoding a LUSTR2 receptor, by microinjection, electroporation, retroviral transfection or other means well known to those skilled in the art, into appropriately fertilized embryos in order to produce a transgenic animal or 2) homologous recombination of mutant or normal, human or animal versions of these genes with the native gene locus in transgenic animals to alter the regulation of expression or the structure of these LUSTR2 receptor sequences. The technique of homologous recombination is well known in the art. It replaces the native gene with the inserted gene and hence is useful for producing an animal that cannot express native LUSTR2 receptors but does express, for example, an inserted mutant LUSTR2 receptor, which has replaced the native LUSTR2 receptor in the animal's genome by recombination, resulting in underexpression of the transporter. Microinjection adds genes to the genome, but does not remove them, and the technique is useful for producing an animal which expresses its own and added LUSTR2 receptor, resulting in overexpression of the LUSTR2 receptor.

One means available for producing a transgenic animal, with a mouse as an example, is as follows: Female mice are mated, and the resulting fertilized eggs are dissected out of their oviducts. The eggs are stored in an appropriate medium such as cesiumchloride M2 medium. DNA or cDNA encoding LUSTR2 is purified from a vector by methods well known to the one skilled in the art. Inducible promoters may be fused with the coding region of the DNA to provide an experimental means to regulate expression of the transgene. Alternatively or in addition, tissue specific regulatory elements may be fused with the coding region to permit tissue-specific expression of the transgene. The DNA, in an appropriately buffered solution, is put into a microinjection needle (which may be made from capillary tubing using a piper puller) and the egg to be injected is put in a depression slide. The needle is inserted into the pronucleus of the egg, and the DNA solution is injected. The injected egg is then transferred into the oviduct of a pseudopregnant mouse which is a mouse stimulated by the appropriate hormones in order to maintain false pregnancy, where it proceeds to the uterus, implants, and develops to term. As noted above, microinjection is not the only method for inserting DNA into the egg but is used here only for exemplary purposes.

## References


U.S. 4,522,811
U.S. 4,683,195
U.S. 4,800,195
U.S. 4,965,188
U.S. 5,223,409
U.S. 5,283,317
U.S. 5,403,484
U.S. 5,565,332
U.S. 5,571,698
U.S. 5,641,673
WO 84/03564
WO 92/01810
WO 93/03151
WO 94/13804
WO 01/04297

Agrawal *et al., Trends Biotechnol. 10,* 152-158, 1992
Altschul SF, Madden TL, Schaffer AA, Zhang J, Zhang Z, Miller W, Lipman DJ; Nucleic Acids Res 1997 Sep 1; 25(17): 3389-402
Appa Rao KB, Garg LC, Panda AK, Totey SM; Protein Expr Purif 1997 Nov; 11(2): 201-8
Barnes, P.J. Mechanisms in COPD. Differences from asthma. Chest 2000, **117**:10S14S
Bartel *et al., BioTechniques 14,* 920-924, 1993
Becker-Andre, M., Meth. Mol. Cell Biol. 2:189-201 (1991)
Botstein D, W. R., Skolnick M, Davis R W., Am J Hum Genet. 32: 314-31, 1980.
Burton, *Proc. Natl. Acad. Sci. 88,* 11120-23, 1991
Carell et al. (1994) Angew. Chem. Int. Ed. Engl. 33:2061; and Gallop et al. (1994) J. Med. Chem. 37:1233
Carrell et al. (1994) Angew. Chem. Int. Ed. Engl. 33:2059
Caruthers *et al., Nucl. Acids Res. Symp. Ser.* 215-223, 1980
Cawkwell L, B. S., Lewis F A, Dixon M F, Taylor G R, Quirke P, Br J Cancer. 67: 1262-7, 1993.
Cech, *Science 236,* 1532-1539; 1987
*Cech, Ann. Rev. Biochem. 59,* 543-568; 1990

Cech, *Curr. Opin. Struct. Biol. 2*, 605-609; 1992
Cho et al. (1993) Science 261:1303
Colbere-Garapin *et al.*, *J. Mol. Biol. 150,* 1-14, 1981
Cole *et al*., *Mol. Cell Biol. 62*, 109-120, 1984)
Coruzzi *et al*., *EMBO J. 3,* 1671-1680, 1984
Cote *et al*., *Proc. Natl. Acad. Sci. 80*, 2026-2030; 1983
Couture & Stinchcomb, *Trends Genet. 12,* 510-515, 1996
Cull et al.(1992) Proc. Natl. Acad. Sci. USA 89:1865-1869
Cwirla et al. (1990) Proc. Natl. Acad. Sci. USA 87:6378-6382
Devlin (1990) Science 249:404-406
DeWitt et al. (1993) Proc. Natl. Acad. Sci. USA 90:6909
Engelhard *et al., Proc. Nat. Acad. Sci. 91,* 3224-3227, 1994
Erb et al. (1994) Proc. Natl. Acad. Sci. USA 91:11422
Felici (1991) J. Mol. Biol. 222:301-310
Fodor (1993) Nature 364:555-556
Gee *et al.,* in Huber & Carr, MOLECULAR AND IMMUNOLOGIC APPROACHES, Futura Publishing Co., Mt. Kisco, N.Y., 1994
Gergen and Weiss, Am Rev Respir Dis 146:823-824, 1992
Gibson, U. E. M., Heid, C. A. and Williams, P. M.., Genome Research. 6: 995-1001, 1996.
Hampton *et al.,* SEROLOGICAL METHODS: A LABORATORY MANUAL, APS Press, St. Paul, Minn., 1990
Hartman & Mulligan, *Proc. Natl. Acad. Sci. 85,* 8047-51, 1988
Haseloff *et al. Nature 334*, 585-591, 1988
Heid, C. A., Stevens, J., Livak, K. J., and Williams, P. M., Genome Research,. 6: 986-994, 1996.
Holland, P. M., Abramson, R. D., Watson, R. and Gelfand, D. H., PNAS. 88: 7276-7280, 1991.
Horn *et al. Nucl. Acids Res. Symp. Ser.* 225-232, 1980
Houghten (1992) Bio/Techniques 13:412-421
Iwabuchi *et al., Oncogene 8,* 1693-1696, 1993
Jeffreys A J, W. V., Thein S L, Nature. 316: 76-9, 1985.
Johnson *et al*., *Endoc. Rev. 10,* 317-331, 1989
Kellogg, D. E., et al., Anal. Biochem. 189:202-208 (1990)
Kohler *et al., Nature 256,* 495-497, 1985
Kozbor *et al., J Immunol. Methods 81,* 31-42, 1985
Kroll *et al., DNA Cell Biol. 12,* 441-453, 1993
Lam (1991) Nature 354:82-84
Lam (1997) Anticancer Drug Des. 12:145
Lefkowitz, *Nature 351,* 353-354, 1991
Livak, K. J., Flood, S. J., Marmaro, J., Giusti, W. and Deetz, K., PCR Methods and Applications 357-362, 1995.
Logan & Shenk, *Proc. Natl. Acad. Sci. 81,* 3655-3659, 1984
Lowy *et al., Cell 22*, 817-23, 1980
Maddox *et al., J. Exp. Med. 158,* 1211-1216, 1983
Madura *et al., J. Biol. Chem. 268,* 12046-12054, 1993
Maniatis et al., Molecular Cloning: A Laboratory Manual, 1982, Cold Spring Harbor Laboratory, Cold Spring Harbor Press
McConnell *et al., Science 257,* 1906-1912, 1992
Merrifield, *J. Am. Chem. Soc. 85,* 2149-2154, 1963
Morrison *et al., Proc. Natl. Acad. Sci. 81,* 6851-6855, 1984 Murray, 1992, *supra*
Nagarenko, I. A., et al. Nucleic Acids Research 25:16-21 (1997)
Neuberger *et al., Nature 312,* 604-608, 1984
Nicholls *et al.,* 1993, *J. Immunol. Meth. 165,* 81-91)
Piatak, M. J., et al., BioTechniques 14:70-81 (1993)
Piatak, M. J., et al., Science 259:1749-1754 (1993))
Porath *et al., Prot. Exp. Purif. 3,* 263-281, 1992
Roberge *et al., Science 269,* 202-204, 1995
Sambrook, J., Fritsch, E. F., and Maniatis, T. (1989) Molecular Cloning: A Laboratory Manual, 2nd ed., Cold Spring Harbor Laboratory Press, NY.
Scott and Smith (1990) Science 249:386-390
Sharp, P. A., et al., Methods Enzymol. 65:750-768, 1980
Sjolander & Urbaniczky, *Anal. Chem. 63,* 2338-2345, 1991

Southern, E. M., J. Mol. Biol., 98:503-517, 1975

Szabo *et al., Curr. Opin. Struct. Biol. 5,* 699-705, 1995

Takamatsu *EMBO J. 6*, 307-311, 1987

Takeda *et al*., *Nature 314,* 452-454, 1985

Thomas, P. S., Proc. Nat. Acad. Sci., 77:5201-5205, 1980)

Uhlmann *et al*., *Chem. Rev. 90,* 543-584, 1990

Uhlmann *et al*., *Tetrahedron. Lett. 215,* 3539-3542, 1987

Verhaar *et al.,* 1995, *Int. J. Cancer 61,* 497-501

Weber et al., Genomics. 7: 524-30, 1990.

Wigler *et al*., *Cell 11,* 223-32, 1977

Wigler *et al., Proc. Natl. Acad. Sci. 77*, 3567-70, 1980

Zervos *et al., Cell 72,* 223-232, 1993

Zuckermann et al. (1994). J. Med. Chem. 37:2678

Lam KS.Application of combinatorial library methods in cancer research and drug discovery.Anticancer Drug Des 1997 Apr;12(3):145-67

## Claims

1. A nucleic acid molecule selected from a group consisting of

   i) nucleic acid molecules encoding a polypeptide comprising the amino acid sequence of SEQ ID NO:2,

   ii) nucleic acid molecules comprising the sequence of SEQ ID NO:1,

   iii) nucleic acid molecules having the sequence of SEQ ID NO:1,

   iv) nucleic acid molecules the complementary strand of which hybridizes under stringent conditions to a nucleic acid molecule of (i), (ii), or (iii); and

   v) nucleic acid molecules the sequence of which differs from the sequence of a nucleic acid molecule of (iii) due to the degeneracy of the genetic code;

   wherein the polypeptide encoded by said nucleic acid molecule has LUSTR2 activity.

2. A purified polypeptide selected from a group consisting of

   i) polypeptides having the sequence of SEQ ID NO:2,

   ii) polypeptides comprising the sequence of SEQ ID NO:2,

   iii) polypeptides encoded by nucleic acid molecules of claim 1; and

   iv) polypeptides which show at least 99%, 98%, 95%, 90%, or 80% homology with a polypeptide of (i), (ii), or (iii);

   wherein said purified polypeptide has LUSTR2 activity.

3. A vector comprising the nucleic acid molecule of claim 1.

4. A host cell containing the vector of claim 3.

5. A method of producing a LUSTR2 comprising the steps of

   i) culturing the host cell of claim 3 under suitable conditions and

   ii) recovering the LUSTR2 from the culture medium.

6. A method for the detection of a polynucleotide encoding a LUSTR2 in a sample comprising the steps of

   i) hybridizing a polynucleotide of claim 1 to nucleic acid material of the sample, thereby forming a hybridization complex; and

   ii) detecting said hybridization complex.

7. The method of claim 6, wherein, before hybridization, the nucleic acid material of the sample is amplified.

8. A method for the detection of a polynucleotide of claim 1 or a polypeptide of claim 2 comprising the steps of

   i) contacting a sample with a reagent which specifically interacts with a polynucleotide of claim 1 or a polypeptide of claim 2; and

   ii) detecting said interaction.

9. A diagnostic kit for conducting the method of any of claims 6 to 8.

**10.** A method for screening for regulators of the activity of a LUSTR2 comprising the steps of

i) contacting a test compound with a polypeptide of claim 2,

ii) detect binding of said test compound to said polypeptide of claim 2,

wherein test compounds that bind under (ii) are identified as potential regulators of the LUSTR2 activity.

**11.** The method of claim 10, wherein the step of contacting is in or at the surface of a cell.

**12.** The method of claim 10 wherein the cell in in vitro.

**13.** The method of claim 10, wherein the step of contacting is in a cell-free system.

**14.** The method of claim 10, wherein the polypeptide is coupled to a detectable label.

**15.** The method of claim 10, wherein the compound is coupled to a detectable label.

**16.** The method of claim 10, wherein the test compound displaces a ligand which is first bound to the polypeptide.

**17.** The method of claim 10, wherein the polypeptide is attached to a solid support.

**18.** The method of claim 10, wherein the compound is attached a solid support.

**19.** A method of screening for regulators of the activity of a LUSTR2 comprising the steps of

i) measuring the activity of a polypeptide of claim 2 at a certain concentration of a test compound or in the absence of said test compound,

ii) measuring the activity of said polypeptide at a different concentration of said test compound,

wherein said test compound is identified as a regulator of the activity of a LUSTR2 when there is a significant difference between the activities measured in (i) and (ii).

**20.** A method of screening for regulators of the activity of a LUSTR2 comprising the steps of

i) measuring the activity of a polypeptide of claim 2 at a certain concentration of a test compound,

ii) measuring the activity of a polypeptide of claim 2 at the presence of a compound known to be a regulator of LUSTR2.

**21.** The method of claim 19 and 20, wherein the activities are measured in a cell.

**22.** The method of claim 19 and 20, wherein the cell is in vitro.

**23.** The method of claim 19 and 20, wherein the activities are measured in a cell-free system.

**24.** A method of screening for regulators of LUSTR2 comprising the steps of

i) contacting a test compound with a nucleic acid molecule of claim 2,

ii) detect binding of said test compound to said nucleic acid molecule,

wherein said test compound is identified as a potential regulator of LUSTR2 when it binds to said nucleic acid molecule.

**25.** The method of claim 24 wherein the nucleic acid molecule is RNA.

**26.** The method of claim 24 wherein the contacting step is in or at the surface of a cell.

**27.** The method of claim 24 wherein the contacting step is in a cell-free system.

**28.** The method of claim 24 wherein the polypeptide or nucleic acid molecule is coupled to a detectable label.

**29.** The method of claim 24 wherein the test compound is coupled to a detectable label.

**30.** A method of regulating the activity of a LUSTR2 wherein LUSTR2 is contacted with a regulator of LUSTR2.

**31.** A method of diagnosing a LUSTR2 related disease in a diseased mammal comprising the steps of

i) measuring the amount of a nucleic acid molecule of claim 1 in a sample taken from said diseased mammal,

ii) comparing the result of (i) to the amount of said nucleic acid molecule in one or several healthy mammals,

wherein a LUSTR2 related disease is diagnosed in the diseased mammal when the amount of said nucleic acid molecule in the diseased mammal is significantly different from the amount of said nucleic acid molecule in the healthy mammal/mammals.

**32.** A pharmaceutical composition comprising a nucleic acid molecule of claim 1.

**33.** A pharmaceutical composition comprising a vector of claim 3.

**34.** A pharmaceutical composition comprising a polypeptide of claim 2.

**35.** A pharmaceutical composition comprising a regulator of any of claims 10 to 31.

**36.** A pharmaceutical composition comprising a regulator of any of claims 10 to 31 for the treatment of hematological disorders, peripheral and central nervous system disorders, COPD, asthma, genito-urological disorders, metabolic diseases, pancreas disorders or heart disorders in a mammal.

**37.** Use of regulators of a LUSTR2 identified by any of claims 10 to 31 for the preparation of a pharmaceutical composition useful for the treatment of hematological disorders, peripheral and central nervous system disorders, COPD, asthma, genito-urological disorders, metabolic diseases, pancreas disorders or heart disorders in a mammal.

**38.** Method for the preparation of a pharmaceutical composition useful for the treatment of hematological disorders, peripheral and central nervous system disorders, COPD, asthma, genito-urological disorders, metabolic diseases, pancreas disorders or heart disorders in a mammal comprising the steps of

i) identifying a regulator of LUSTR2 by a method of any of claims 10 to 31

ii) determining whether said regulator ameliorates the symptoms of hematological disorders, peripheral and central nervous system disorders, COPD, asthma, genito-urological disorders, metabolic diseases, pancreas disorders or heart disorders in a mammal.

iii) combining of said regulator with an acceptable pharmaceutical carrier.

**39.** Use of a regulator of LUSTR2 identified by any of claims 10 to 31 for the treatment of hematological disorders, peripheral and central nervous system disorders, COPD, asthma, genito-urological disorders, metabolic diseases, pancreas disorders or heart disorders in a mammal.

**40.** Use of a regulator of LUSTR2 identified by any of claims 10 to 31 for the regulation of LUSTR2 activity in a mammal having a hematological disorder, peripheral and central nervous system disorder, COPD, asthma, a genito-urological disorder, a metabolic disease, a pancreas disorder or a heart disorder.

**41.** Use of a pharmaceutical composition according to any of claims 35 to 40,

wherein the regulator is

i) a small molecule,
ii) an RNA molecule,
iii) an antisense oligonucleotide,
iv) a polypeptide,
v) an antibody, or
vi) a ribozyme.
and/or activate or inhibit the activity of LUSTR2 as well as pharmaceutical compositions comprising such compounds.

**Fig. 1**

**SEQ ID NO:1**

ATGGCAGTGAGCGAGAGGAGGGGGCTCGGCCGCGGGAGCCCCGCGGAGTGGGGG
CAGCGGCTACTTCTGGTGCTGCTGTTGGGTGGCTGCTCCGGGCGCATCCACCGG
CTGGCGCTGACGGGGGAGAAGCGAGCGGACATCCAGCTGAACAGCTTCGGTTTC
TACACCAATGGCTCTCTGGAGGTGGAGTTGAGCGTCCTGCGGCTGGGCCTCCGG
GAGGCAGAAGAGAAGTCCCTGCTGGTGGGGTTCAGTCTCAGCCGGGTTCGGTCT
GGCAGAGTTCGCTCCTATTCAACCCGGGATTTCCAGGACTGCCCTCTCCAGAAA
AACAGTAGCAGTTTCCTGGTCCTGTTCCTCATCAACACCAAGGATCTGCAGGTC
CAGGTGCGGAAGTATGGAGAGCAGAAGACGTTGTTTATCTTTCCCGGGCTCCTC
CCGGAAGCACCCTCCAAACCAGGGCTCCCGAAGCCACAGGCCACAGTCCCCCGC
AAGGTGGATGGCGGAGGGACCTCTGCAGCCAGCAAGCCCAAGTCAACACCCGCA
GTGATTCAGGGTCCTAGTGGGAAGGACAAGGACCTGGTGTTGGGCCTGAGCCAC
CTCAACAACTCCTACAACTTCAGTTTCCACGTGGTGATCGGCTCTCAGGCGGAA
GAAGGCCAGTACAGCCTGAACTTCCACAACTGCAACAATTCAGTGCCAGGAAAG
GAGCATCCATTCGACATCACGGTGATGATCCGGGAGAAGAACCCCGATGGCTTC
CTGTCGGCAGCGGAGATGCCCCTTTTCAAGCTCTACATGGTCATGTCCGCCTGC
TTCCTGGCCGCTGGCATCTTCTGGGTGTCCATCCTCTGCAGGAACACGTACAGC
GTCTTCAAGATCCACTGGCTCATGGCGGCCTTGGCCTTCACCAAGAGCATCTCT
CTCCTCTTCCACAGCATCAACTACTACTTCATCAACAGCCAGGGCCACCCCATC
GAAGGCCTTGCCGTCATGTACTACATCGCACACCTGCTGAAGGGCGCCCTCCTC
TTCATCACCATCGCCCTGATTGGCTCAGGCTGGGCCTTCATCAAGTACGTCCTG
TCGGATAAGGAGAAGAAGGTCTTTGGGATCGTGATCCCCATGCAGGTCCTGGCC
AACGTGGCCTACATCATCATCGAGTCCCGCGAGGAAGGCGCCAGCGACTACGTG
CTGTGGAAGGAGATTTTGTTCCTGGTGGACCTCATCTGCTGTGGTGCCATCCTG
TTCCCCGTAGTCTGGTCCATCCGGCATCTCCAGGATGCGTCTGGCACAGACGGG
AAGGTGGCAGTGAACCTGGCCAAGCTGAAGCTGTTCCGGCATTACTATGTCATG
GTCATCTGCTACGTCTACTTCACCCGCATCATCGCCATCCTGCTGCAGGTGGCT
GTGCCCTTTCAGTGGCAGTGGCTGTACCAGCTCTTGGTGGAGGGCTCCACCCTG

GCCTTCTTCGTGCTCACGGGCTACAAGTTCCAGCCCACAGGAAACAACCCGTAC
CTGCAGCTGCCCCAGGAGGACGAGGAGGATGTTCAGATGGAGCAAGTAATGACG
GACTCTGGGTTCCGGGAAGGCCTCTCCAAAGTCAACAAAACAGCCAGCGGGCGG
GAACTGTTATGA

**Fig. 2**

**SEQ ID NO:2**

mavserrglgrgspaewgqrlllvlllggcsgrihrlaltgekradiqlnsfgf
ytngslevelsvlrlglreaeeksllvgfslsrvrsgrvrsystrdfqdcplqk
nsssflvlflintkdlqvqvrkygeqktlfifpgllpeapskpglpkpqatvpr
kvdgggtsaaskpkstpaviqgpsgkdkdlvlglshlnnsynfsfhvvigsqae
egqyslnfhncnnsvpgkehpfditvmireknpdgflsaaemplfklymvmsac
flaagifwvsilcrntysvfkihwlmaalaftksisllfhsinyyfinsqghpi
eglavmyyiahllkgallfitialigsgwafikyvlsdkekkvfgivipmqvla
nvayiiiesreegasdyvlwkeilflvdliccgailfpvvwsirhlqdasgtdg
kvavnlaklklfrhyyvmvicyvyftriiaillqvavpfqwqwlyqllvegstl
affvltgykfqptgnnpylqlpqedeedvqmeqvmtdsgfreglskvnktasgr
ell

**Fig. 3**

**SEQ ID NO:3**

5`-GGGTTCGGTCTGGCAGAGT-3`

**Fig. 4**

**SEQ ID NO:4**

5`-CGCTCCTATTCAACCCGGGATTTCC-3`

**Fig. 5**

**SEQ ID NO:5**

5`-TTTTTCTGGAGAGGGCAGTCC-3`

European Patent Office

# PARTIAL EUROPEAN SEARCH REPORT

which under Rule 45 of the European Patent Convention shall be considered, for the purposes of subsequent proceedings, as the European search report

Application Number EP 01 12 7439

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | WO 01 04297 A (KATO SEISHI ;KIMURA TOMOKO (JP); PROTEGENE INC (JP); SAGAMI CHEM R) 18 January 2001 (2001-01-18)<br>* SEQ ID NO: 10; SEQ ID NO: 30; SEQ ID NO: 20 *<br>* page 6-47; claims 1-7; examples 1-5 * | 1-41 | C12N15/12<br>C12N1/21<br>C12N5/10<br>C12N15/63<br>C07K14/705<br>C12Q1/68<br>G01N33/68<br>A61K31/7088<br>A61K31/713<br>A61K38/17<br>A61K39/395<br>A61K48/00 |
| X | WO 01 34800 A (HUMAN GENOME SCIENCES INC ;EBNER REINHARD (US); RUBEN STEVEN M (US) 17 May 2001 (2001-05-17)<br>* SEQ ID NO: 84; SEQ ID NO: 120; SEQ ID NO: 29; SEQ ID NO: 65 *<br>* claims 1-23; examples 1-58 * | 1-41 | |
| X | WO 99 33870 A (INCYTE PHARMA INC ;CORLEY NEIL C (US); YUE HENRY (US); AU YOUNG JA) 8 July 1999 (1999-07-08)<br>* SEQ ID NO: 7; SEQ ID NO: 19 *<br>* page 17-53; claims 1-20; examples 1-12 * | 1-41 | |
| | -/-- | | TECHNICAL FIELDS SEARCHED (Int.Cl.7)<br>C12N<br>C07K<br>C12Q<br>G01N<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 8 May 2002 | Devijver, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

INCOMPLETE SEARCH SHEET C

Application Number
EP 01 12 7439

Although claim 31 (as far as in vivo methods are concerned) is directed to a diagnostic method practised on the human/animal body (Article 52(4) EPC), the search has been carried out and based on the alleged effects of the compound/composition.
Although claims 30, 39-41 (all in part and as far as in vivo methods are concerned) are directed to a method of treatment of the human/animal body (Article 52(4) EPC), the search has been carried out and based on the alleged effects of the compound/composition.

---

Claim(s) searched incompletely:
8,9,20-23,30,35-41

Reason for the limitation of the search:

Claims 8 and 9 refer to a reagent which specifically interacts with a polynucleotide of claim 1 or a polypeptide of claim 2 without giving a true technical characterization. The claims cover all compounds having this characteristic or property, whereas the application provides support within the meaning of Article 84 EPC and/or disclosure within the meaning of Article 83 EPC for only a very limited number of such compounds, namely: antibodies which specifically bind to a polypeptide of claim 2, antisense (complementary) oligonucleotides of claim 1 and ribozymes. In consequence, the scope of said claims is ambiguous and vague, and their subject-matter is not sufficiently disclosed and supported (Art. 83 and 84 EPC). An attempt is made to define the compound by reference to a result to be achieved.
This lack of clarity in the present case is such as to render a meaningful search over the whole of the claimed scope impossible. Consequently, the search has been carried out for those parts of the claims which appear to be clear, supported and disclosed, namely those parts relating to: antibodies which specifically bind to a polypeptide of claim 2, antisense (complementary) oligonucleotides of claim 1 and ribozymes.
The above comment also applies for the regulator referred to in claims 20-23, 30, 35-41.

European Patent Office

# PARTIAL EUROPEAN SEARCH REPORT

Application Number
EP 01 12 7439

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| D,X | DATABASE EMBL 'Online!<br>11 July 2000 (2000-07-11)<br>CARIM L ET AL: "Homo sapiens mRNA full length insert cDNA clone EUROIMAGE 1703145."<br>Database accession no. AL365404<br>XP002196205<br>--- | 1-8 | |
| D,X | DATABASE EMBL 'Online!<br>1 June 2001 (2001-06-01)<br>EDGAR A J: "Mus musculus lung seven transmembrane receptor 2 (Lustr2) mRNA, complete cds."<br>Database accession no. AF376726<br>XP002196206<br>--- | 1-8 | TECHNICAL FIELDS SEARCHED (Int.Cl.7) |
| A | DATABASE EMBL 'Online!<br>1 June 2001 (2001-06-01)<br>EDGAR A J: "Homo sapiens lung seven transmembrane receptor 1 (LUSTR1) mRNA, complete cds."<br>Database accession no. AF376725<br>XP002196207<br>--- | | |
| A | LEE D K ET AL: "Cloning and characterization of additional members of the G protein-coupled receptor family"<br>BIOCHIMICA ET BIOPHYSICA ACTA. GENE STRUCTURE AND EXPRESSION, ELSEVIER, AMSTERDAM, NL,<br>vol. 1490, no. 3,<br>29 February 2000 (2000-02-29), pages 311-323, XP004275600<br>ISSN: 0167-4781<br>---<br>-/-- | | |

EPO FORM 1503 03.82 (P04C10)

European Patent Office

# PARTIAL EUROPEAN SEARCH REPORT

Application Number
EP 01 12 7439

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | LIBERT F ET AL: "SELECTIVE AMPLIFICATION AND CLONING OF FOUR NEW MEMBERS OF THE G PROTEIN-COUPLED RECEPTOR FAMILY" SCIENCE, AMERICAN ASSOCIATION FOR THE ADVANCEMENT OF SCIENCE,, US, vol. 244, no. 4904, 5 May 1989 (1989-05-05), pages 569-572, XP000073306 ISSN: 0036-8075 ----- | | |
| | | | TECHNICAL FIELDS SEARCHED (Int.Cl.7) |

EPO FORM 1503 03.82 (P04C10)

## ANNEX TO THE EUROPEAN SEARCH REPORT ON EUROPEAN PATENT APPLICATION NO. EP 01 12 7439

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2002

| Patent document cited in search report | Publication date | Patent family member(s) | Publication date |
|---|---|---|---|
| WO 0104297 A | 18-01-2001 | AU 5249700 A | 30-01-2001 |
| | | EP 1200582 A2 | 02-05-2002 |
| | | WO 0104297 A2 | 18-01-2001 |
| WO 0134800 A | 17-05-2001 | AU 1916101 A | 06-06-2001 |
| | | WO 0134800 A1 | 17-05-2001 |
| WO 9933870 A | 08-07-1999 | AU 2205499 A | 19-07-1999 |
| | | CA 2316079 A1 | 08-07-1999 |
| | | EP 1042362 A2 | 11-10-2000 |
| | | JP 2001526914 T | 25-12-2001 |
| | | WO 9933870 A2 | 08-07-1999 |

EPO FORM P0459

For more details about this annex : see Official Journal of the European Patent Office, No. 12/82